# EUROPEAN PATENT SPECIFICATION

(11) **EP 1 124 804 B1**
(45) Date of publication and mention of the grant of the patent: **24.08.2005**
(21) Application number: 99953259.1
(22) Date of filing: 27.10.1999
(51) Int. Cl.: C07D 237/14, A61K 31/50, C07D 405/04, C07D 409/04

(54) **PROSTAGLANDIN ENDOPEROXIDE H SYNTHASE BIOSYNTHESIS INHIBITORS**
PROSTAGLANDIN ENDOPEROXYDE H SYNTHASE BIOSYNTHESE INHIBITOREN
INHIBITEURS DE LA BIOSYNTHESE DE LA PROSTAGLANDINE ENDOPEROXYDE H SYNTHASE

(30) Priority: 27.10.1998 US 179605; 03.03.1999 US 261872; 23.04.1999 US 298490; 27.10.1999 US 427768
(43) Date of publication of application: 22.08.2001
(73) Proprietor: ABBOTT LABORATORIES, IL 60064-6050 (US)
(72) Inventor: BLACK, Lawrence, A., Libertyville, IL 60048 (US); BASHA, Anwer, Lake Forest, IL 60045 (US); KOLASA, Teodozyj, Lake Villa, IL 60046 (US); KORT, Michael, E., Lake Bluff, IL 60044 (US); LIU, Huaqing, Buffalo Grove, IL 60089 (US); MCCARTY, Catherine, M., Highland Park, IL 60035 (US); PATEL, Meena, V., Green Oaks, IL 60048 (US); ROHDE, Jeffrey, J., Evanston, IL 60202 (US); COGHLAN, Michael, J., Grayslake, IL 60030 (US); STEWART, Andrew, O., Libertyville, IL 60048 (US)
(74) Representative: Modiano, Guido
(86) International application number: PCT/US1999/025234
(87) International publication number: WO 2000/024719

(56) References cited:
- WO-A-98/41511
- WO-A-99/10331
- WO-A-99/10332

## Description

### TECHNICAL FIELD

The present invention encompasses novel pyridazinone compounds useful in the treatment of cyclooxygenase-2 mediated diseases. More particularly, the compounds inhibit prostaglandin biosynthesis, particularly the induced prostaglandin endoperoxide H synthase (PGHS-2, cyclooxygenase-2, COX-2) protein.

### BACKGROUND OF THE INVENTION

The prostaglandins are extremely potent substances which produce a wide variety of biological effects, often in the nanomolar to picomolar concentration range. The discovery of two forms of prostaglandin endoperoxide H synthase, isoenzymes PGHS-1 and PGHS-2, that catalyze the oxidation of arachidonic acid leading to prostaglandin biosynthesis has resulted in renewed research to delineate the role of these two isozymes in physiology and pathophysiology. These isozymes have been shown to have different gene regulation and represent distinctly different prostaglandin biosynthesis pathways. The PGHS-1 pathway is expressed constitutively in most cell types. It responds to produce prostaglandins that regulate acute events in vascular homeostasis and also has a role in maintaining normal stomach and renal function. The PGHS-2 pathway involves an induction mechanism which has been linked to inflammation, mitogenesis and ovulation phenomena.

Prostaglandin inhibitors provide therapy for pain, fever, and inflammation, and are useful therapies, for example in the treatment of rheumatoid arthritis and osteoarthritis. The non-steroidal anti-inflammatory drugs (NSAIDs) such as ibuprofen, naproxen and fenamates inhibit both isozymes. Inhibition of the constitutive enzyme PGHS-1 results in gastrointestinal side effects including ulcers and bleeding and incidence of renal problems with chronic therapy. Inhibitors of the induced isozyme PGHS-2 may provide anti-inflannmatory activity without the side effects of PGHS-1 inhibitors.

The problem of side-effects associated with NSAID administration has never completely been solved in the past. Enteric coated tablets and co-administration with misoprostol, a prostaglandin derivative, have been tried in an attempt to minimize stomach toxicity. It would be advantageous to provide compounds which are selective inhibitors of the induced isozyme PGHS-2.

The present invention discloses novel compounds which are selective inhibitors of PGHS-2.

### SUMMARY OF THE INVENTION

The present invention discloses pyridazinone compounds which are selective inhibitors of cyclooxygenase-2 (COX-2). Compounds of the present invention may be selected from the group consisting of
2-(4-Fluorophenyl)-4-(4-hydroxy-2-methyl-1-butoxy)-5-[4-(methylsulfonyl)phenyl]-3(2H)-pyridazinone;
2-(4-Fluorophenyl)-4-(3-hydroxy-3-methyl-1-butoxy)-5-[4-(aminosulfonyl)phenyl]-3(2H)-pyridazinone;
2-(3,4-Difluorophenyl)-4-(3-hydroxy-3-methyl-1-butoxy)-5-[4-(methylsulfonyl)phenyl]-3(2H)-pyridazinone;
2-(3,4-Difluorophenyl)-4-(3-hydroxy-3-methyl-1-butoxy)-5-[4-(aminosulfonyl)phenyl]-3(2H)-pyridazinone;
2-(3-Chloro-4-fluorophenyl)-4-(3-hydroxy-3-methyl-1-butoxy)-5-[4-(methylsulfonyl)phenyl]-3(2H)-pyridazinone;
2-(3-Chloro-4-fluorophenyl)-4-(3-hydroxy-3-methyl-1-butoxy)-5-[4-(aminosulfonyl)phenyl]-3(2H)-pyridazinone;
2-(3-Chlorophenyl)-4-(3-hydroxy-3-methyl-1-butoxy)-5-[4-(methylsulfonyl)phenyl]-3(2H)-pyridazinone;
2-(3-Chlorophenyl)-4-(3-hydroxy-3-methyl-1-butoxy)-5-[4-(aminosulfonyl)phenyl]-3(2H)-pyridazinone;
2-(4-Fluorophenyl)-4-(2-hydroxy-2-methyl-1-propoxy)-5-[4-(methylsulfonyl)phenyl]-3(2H)-pyridazinone;
2-(4-Fluorophenyl)-4-(2-hydroxy-2-methyl-1-propoxy)-5-[4-(aminosulfonyl)phenyl]-3(2H)-pyridazinone;
2-(3-Chloro-4-fluorophenyl)-4-(2-hydroxy-2-methyl-1-propoxy)-5-[4-(methylsulfonyl)phenyl]-3(2H)-pyridazinone;
2-(3-Chloro-4-fluorophenyl)-4-(2-hydroxy-2-methyl-1-propoxy)-5-[4-(aminosulfonyl)phenyl]-3(2H)-pyridazinone;
2-(3-Chlorophenyl)-4-(2-hydroxy-2-methyl-1-propoxy)-5-[4-(methylsulfonyl)phenyl]-3(2H)-pyridazinone;
2-(3-Chlorophenyl)-4-(2-hydroxy-2-methyl-1-propoxy)-5-[4-(aminosulfonyl)phenyl]-3(2H)-pyridazinone;
2-(4-Fluorophenyl)-4-(4-hydroxy-3-methyl-1-butoxy)-5-[4-(aminosulfonyl)phenyl]-3(2H)-pyridazinone;
2-(3,4-Difluorophenyl)-4-(4-hydroxy-3-methyl-1-butoxy)-5-[4-(methylsulfonyl)phenyl]-3(2H)-pyridazinone;
2-(3-Chloro-4-fluorophenyl)-4-(4-hydroxy-3-methyl-1-butoxy)-5-[4-(methylsulfonyl)phenyl]-3(2H)-pyridazinone;
2-(3-Chlorophenyl)-4-(4-hydroxy-3-methyl-1-butoxy)-5-[4-(methylsulfonyl)phenyl]-3(2H)-pyridazinone;
2-(3,4-Difluorophenyl)-4-(4-hydroxy-3-methyl-1-butoxy)-5-[4-(aminosulfonyl)phenyl]-3(2H)-pyridazinone;
2-(3-Chloro-4-fluorophenyl)-4-(4-hydroxy-3-methyl-1-butoxy)-5-[4-(aminosulfonyl)phenyl]-3(2H)-pyridazinone;
2-(3-Chlorophenyl)-4-(4-hydroxy-3-methyl-1-butoxy)-5-[4-(aminosulfonyl)phenyl]-3(2H)-pyridazinone;
(S)-2-(4-Fluorophenyl)-4-(3-hydroxy-2-methyl-1-propoxy)-5-[4-(aminosulfonyl)phenyl]-3(2H)-pyridazinone;
(R)-2-(4-Fluorophenyl)-4-(3-hydroxy-2-methyl-1-propoxy)-5-[4-(aminosulfonyl)phenyl]-3(2H)-pyridazinone;
(S)-2-(3-Chloro-4-fluorophenyl)-4-(3-hydroxy-2-methyl-1-propoxy)-5-[4-(methylsulfonyl)phenyl]-3(2H)-pyridazinone;
(S)-2-(3-Chloro-4-fluorophenyl)-4-(3-hydroxy-2-methyl-1-propoxy)-5-[4-(aminosulfonyl)phenyl]-3(2H)-pyridazinone;
(R)-2-(3-Chloro-4-fluorophenyl)-4-(3-hydroxy-2-methyl-1-propoxy)-5-[4-(methylsulfonyl)phenyl]-3(2H)-pyridazinone;
(R)-2-(3-Chloro-4-fluorophenyl)-4-(3-hydroxy-2-methyl-1-propoxy)-5-[4-(aminosulfonyl)phenyl]-3(2H)-pyridazinone;
(S)-2-(3-Chlorophenyl)-4-(3-hydroxy-2-methyl-1-propoxy)-5-[4-(methylsulfonyl)phenyl]-3(2H)-pyridazinone;
(S)-2-(3-Chlorophenyl)-4-(3-hydroxy-2-methyl-1-propoxy)-5-[4-(aminosulfonyl)phenyl]-3(2H)-pyridazinone;
(R)-2-(3-Chlorophenyl)-4-(3-hydroxy-2-methyl-1-propoxy)-5-[4-(methylsulfonyl)phenyl]-3(2H)-pyridazinone;
(R)-2-(3-Chlorophenyl)-4-(3-hydroxy-2-methyl-1-propoxy)-5-[4-(aminosulfonyl)phenyl]-3(2H)-pyridazinone;
2-(4-Fluorophenyl)-4-(3-hydroxy-2,2-dimethyl-1-propoxy)-5-[4-(methylsulfonyl)phenyl]-3(2H)-pyridazinone;
2-(4-Fluorophenyl)-4-(3-hydroxy-2,2-dimethyl-1-propoxy)-5-[4-(aminosulfonyl)phenyl]-3(2H)-pyridazinone;
2-(3,4-Difluorophenyl)-4-(3-hydroxy-2,2-dimethyl-1-propoxy)-5-[4-(methylsulfonyl)phenyl]-3(2H)-pyridazinone;
2-(3,4-Difluorophenyl)-4-(3-hydroxy-2,2-dimethyl-1-propoxy)-5-[4-(aminosulfonyl)-phenyl]-3(2H)-pyridazinone;
2-(3-Chloro-4-fluorophenyl)-4-(3-hydroxy-2,2-dimethyl-1-propoxy)-5-[4-(methylsulfonyl)phenyl]-3(2H)-pyridazinone;
2-(3-Chloro-4-fluorophenyl)-4-(3-hydroxy-2,2-dimethyl-1-propoxy)-5-[4-(aminosulfonyl)phenyl]-3(2H)-pyridazinone;
2-(3-Chlorophenyl)-4-(3-hydroxy-2,2-dimethyl-1-propoxy)-5-[4-(methylsulfonyl)phenyl]-3(2H)-pyridazinane;
2-(3-Chlorophenyl)-4-(3-hydroxy-2,2-dimethyl-1-propoxy)-5-[4-(aminosulfonyl)phenyl]-3(2H)-pyridazinone;
2-(3,4-Dichlorophenyl)-4-(3-hydroxy-3-methyl-1-butoxy)-5-[4-(methylsulfonyl)phenyl]-3(2H)-pyridazinone;
2-[(3-Trifluoromethyl)phenyl]-4-(2-hydroxy-2-methyl-1-propoxy)-5-[4-(methylsulfonyl)phenyl]-3(2H)-pyridazinone;
2-(3,4-Dichlorophenyl)-4-(2-hydroxy-2-methyl-1-propoxy)-5-[4-(methylsulfonyl)phenyl]-3(2H)-pyridazinone; and
2-[3-(Trifluoromethyl)phenyl]-4-(3-hydroxy-3-methyl-1-butoxy)-5-[4-(methylsulfonyl)phenyl]-3(2H)-pyridazinone;
or a pharmaceutically acceptable salt or ester thereof.

Compounds of the invention may also be selected from the group consisting of
2-(2,2,2-Trifluoroethyl)-4-(2-hydroxy-2-methyl-1-propoxy)-5-[4-(methylsulfonyl)phenyl]-3(2H)-pyridazinone;
2-(2,2,2-Trifluoroethyl)-4-(2-hydroxy-2-methyl-1-propoxy)-5-[4-(aminosulfonyl)phenyl]-3(2H)-pyridazinone;
2-(2,2,2-Trifluoroethyl)-4-(3-hydroxy-2,2-dimethyl-1-propoxy)-5-[4-(methylsulfonyl)phenyl]-3(2H)-pyridazinone;
2-(2,2,2-Trifluoroethyl)-4-(3-hydroxy-2,2-dimethyl-1-propoxy)-5-[4-(aminosulfonyl)phenyl]-3(2H)-pyridazinone;
(S)-2-(2,2,2-Trifluoroethyl)-4-(3-hydroxy-2-methyl-1-propoxy)-5-[4-(methylsulfonyl)phenyl]-3(2H)-pyridazinone;
(S)-2-(2,2,2-Trifluoroethyl)-4-(3-hydroxy-2-methyl-1-propoxy)-5-[4-(aminosulfonyl)phenyl]-3(2H)-pyridazinone;
(R)-2-(2,2,2-Trifluoroethyl)-4-(3-hydroxy-2-methyl-1-propoxy)-5-[4-(methylsulfonyl)phenyl]-3(2H)-pyridazinone;
(R)-2-(2,2,2-Trifluoroethyl)-4-(3-hydroxy-2-methyl-1-propoxy)-5-[4-(aminosulfonyl)phenyl]-3(2H)-pyridazinone;
2-(2,2,2-Trifluoroethyl)-4-(3-hydroxy-3-methyl-1-butoxy)-5-[4-(methylsulfonyl)phenyl]-3(2H)-pyridazinone; and
2-(2,2,2-Trifluoroethyl)-4-(3-hydroxy-3-methyl-1-butoxy)-5-[4-(aminosulfonyl)phenyl]-3(2H)-pyridazinone;
or a pharmaceutically acceptable salt or ester thereof.

Compounds of the invention may furthermore be selected from the group consisting of
N-[[4-[2-(4-Fluorophenyl)-4-(3-hydroxy-3-methyl-1-butoxy)-2H-pyridazin-3-on-5-yl]phenyl]sulfonyl]acetamide;
N-[[4-[2-(4-Fluorophenyl)-4-(3-hydroxy-3-methyl-1-butoxy)-2H-pyridazin-3-on-5-yl]phenyllsulfonyl]acetamide, sodium salt;
N-[[4-[2-(3,4-Difluorophenyl)-4-(2-hydroxy-2-methyl-1-propoxy)-2H-pyridazin-3-on-5-yl]phenyl]sulfonyl]acetamide;
N-[[4-[2-(3,4-Difluorophenyl)-4-(2-hydroxy-2-methyl-1-propoxy)-2H-pyridazin-3-on-5-yl]phenyl]sulfonyl]acetamide, sodium salt;
N-[[4-[2-(3-Chloro-4-fluorophenyl)-4-(2-hydroxy-2-methyl-1-propoxy)-2H-pyridazin-3-on-5-yl]phenyl]sulfonyl]acetamide;
N-[[4-[2-(3-Chloro-4-fluorophenyl)-4-(2-hydroxy-2-methyl-1-propoxy)-2H-pyridazin-3-on-5-yl]phenyl]sulfonyl]acetamide, sodium salt;
N-[[4-[2-(3-Chlorophenyl)-4-(2-hydroxy-2-methyl-1-propoxy)-2H-pyridazin-3-on-5-yl]phenyl]sulfonyl]acetamide;
N-[[4-[2-(3-Chlorophenyl)-4-(2-hydroxy-2-methyl-1-propoxy)-2H-pyridazin-3-on-5-yl]phenyl]sulfonyl]acetamide, sodium salt;
N-[[4-[2-(2,2,2-Trifluoroethyl)-4-(2-hydroxy-2-methyl-1-propoxy)-2H-pyridazin-3-on-5-yl]phenyl]sulfonyl]acetamide;
N-[[4-[2-(2,2,2-Trifluoroethyl)-4-(2-hydroxy-2-methyl-1-propoxy)-2H-pyridazin-3-on-5-yl]phenyl]sulfonyl]acetamide, sodium salt;
N-[[4-[2-(2,2,2-Trifluoroethyl)-4-(3-hydroxy-2,2-dimethyl-1-propoxy)-2H-pyridazin-3-on-5-yl]phenyl]sulfonyl]acetamide; and
N-[[4-[2-(2,2,2-Trifluoroethyl)-4-(3-hydroxy-2,2-dimethyl-1-propoxy)-2H-pyridazin-3-on-5-yl]phenyl]sulfonyl]acetamide, sodium salt;
or a pharmaceutically acceptable salt or ester thereof

Another embodiment of the present invention relates to pharmaceutical compositions comprising a therapeutically effective amount of one of the above compounds or a pharmaceutically acceptable salt or ester thereof in combination with a pharmaceutically acceptable carrier for inhibiting prostaglandin biosynthesis.

The compounds of the invention are useful in a method of inhibiting prostaglandin biosynthesis comprising administering a therapeutically effective amount of the compound or a pharmaceutically acceptable salt or ester thereof.

The compounds of the invention are useful in a method of treating pain, fever, inflammation, rheumatoid arthritis, osteoarthritis, adhesions, and cancer comprising administering a therapeutically effective amount of the compound or a pharmaceutically acceptable salt or ester thereof.

A preferred embodiment of the present invention relates to 2-(3,4-difluorophenyl)-4-(3-hydroxy-3-methyl-1-butoxy)-5-[4-(methylsulfonyl)phenyl]-3(2H)-pyridazinone EMBEDor a pharmaceutically acceptable salt or ester thereof.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrabromide, hydroiodide, 2-hydroxy-ethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid.

Basic addition salts can be prepared in situ during the final isolation and purification of the compounds of the invention, or separately by reacting a carboxylic acid function with a suitable base such as the hydroxide, carbonate or bicarbonate of a pharmaceutically acceptable metal cation or with ammonia, or an organic primary, secondary or tertiary amine. Such pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like.

The term "pharmaceutically acceptable ester" as used herein refers to esters which hydrolyze in vivo and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyates, acrylates and ethylsuccinates.

The term "pharmaceutically acceptable prodrug" as used herein refers to those prodrugs of the compounds of the present invention which are, within the scope of sound medical judgement, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed in vivo to provide the parent compound having the invention, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

Asymmetric centers may exist in the compounds of the present invention. The present invention contemplates the various stereoisomers and mixtures thereof. Individual stereoisomers of compounds of the present invention are made by synthesis from starting materials containing the chiral centers or by preparation of mixtures of enantiomeric products followed by separation as, for example, by conversion to a mixture of diastereomers followed by separation by recrystallization or chromatographic techniques, or by direct separation of the optical enantiomers on chiral chromatographic columns. Starting compounds of particular stereochemistry are either commercially available or are made by the methods detailed below and resolved by techniques well known in the organic chemical arts.

The present invention discloses pyridazinone compounds which are cyclooxygenase (COX) inhibitors and are selective inhibitors of cyclooxygenase-2 (COX-2). COX-2 is the inducible isoform associated with inflammation, as opposed to the constitutive isoform, cyclooxygenase-1 (COX-1) which is an important "housekeeping" enzyme in many tissues, including the gastrointestinal (GI) tract and the kidneys.

### Preparation of Compounds of the Invention

### Abbreviations

As used throughout this specification, the following abbreviations have been used: ACD for acid citrate dextrose, CAP for carrageenan induced air pouch prostaglandin, COX-2 for cyclooxygenase-2, CPE for carrageenan induced paw edema in rats, DBAD for di-t-butylazodicarboxylate, DEAD for diethyl azodicarboxylate, DIAD for disopropylazodicarboxylate, DMAP for 4-(dimethylamino)pyridine, DME for 1,2-dimethoxyethane, DMF for N,N-dimethylformamide, DMSO for dimethyl sulfoxide, DMSO for dimethyl sulfoxide, EDTA for ethylenediaminetetraacetic acid, EIA for enzyme immunoassay, FAB for fast atom bombardment, GI for gastrointestinal, HMDS, lithium or Li HMDS for lithium 1,1,1,3,3,3-hexamethyldisilazide, HWPX for Human Whole Platelet Cyclooxygenase-1, MCPBA for meta-chloroperoxybenzoic acid, NSAIDs for non-steroidal anti-inflammatory drugs, PEG 400 for polyethyleneglycol, PGE₂ for prostaglandin E₂, PGHS for prostaglandin endoperoxide H synthase, RHUCX1 for recombinant human cyclooxygenase-1, RHLTCX2 for recombinant human cyclooxygenase-2, r-hu Cox1 for recombinant human Cox-1, TEA for triethylamine, TFA for trifluoroacetic acid, and THF for tetrahydrofuran and WISH for human amnionic whole cell cyclooxygenase-2.

### Example 1 (reference example)

### 2-Benzyl-4-(4-fluorophenyl)-5-methoxy-3(2H)-pyridazinone

To a mixture of 2-benzyl-5-methoxy-4-bromo-3(2H)-pyridazinone, prepared according to the method of (S. Cho et al. described in J. Het. Chem., (1996),33, 1579-1582), (2.94 g; 10 mmol), 4-fluorobenzeneboronic acid (1.54 g; 11 mmol), and CsF (3.04 g; 22 mmol) in 25 mL of anhydrous DME, under N₂, was added Pd(Ph₃P)₄ (347 mg 0.3 mmol). After addition, the mixture was heated at reflux for at 100 °C, for 18 hours. The mixture was concentrated in vacuo and the residue partitioned between ethyl acetate and water. The acetate layer was washed with brine, dried over MgSO₄ and concentrated in vacuo. The solid residue was suspended in ethyl ether-hexanes and filtered to provide a solid product (yield: 3.1 g; about 100%; > 95% purity). ¹H NMR (300 MHz, CDCl₃) δ 3.90 (s, 3H), 5.36 (s, 2H), 7.09 (t, J=9 Hz, 2H), 7.31 (m, 3H), 7.50 (m, 4H), 7.91 (s, 1H). MS (DCI/NH₃) m/z 311 (M+H)⁺, 328 (M+NH₄)⁺.

### Example 2 (reference example)

### 2-Benzyl-4-(4-fluorophenyl)-5-hydroxy-3(2H)-pyridazinone

The product from Example 1 (1.24 g; 4 mmol) in 20 mL of acetic acid was treated with aqueous 48% HBr (25 mL). The mixture was heated at reflux for about 5 to about 8 hours (TLC analysis). The mixture was concentrated in vacuo. The product was dissolved in ethyl acetate, washed with 10% bicarbonate, brine and concentrated in vacuo. The residue was treated with diethyl ether-hexanes (2:1) and the solid was filtered to provide an almost pure product (yield: 1.16 g; 98%). ¹H NMR (300 MHz, DMSO-d₆) δ 5.24 (2H), 7.21 (m, 2H), 7.30 (m, 5H), 7.55 (m, 2H), 7.85 (s, 1H), 11.31 (br s, 1H). MS (DCI/NH₃) m/z 296 (M+H)⁺, 314 (M+NH₄)⁺.

### Example 3 (reference example)

### 2-Benzyl-4-(4-fluorophenyl)-5-(trifluoromethylsulfonyloxy)-3(2H)-pyridazinone

A solution of the product from Example 2, (89 mg, 0.3 mmol) in 2.5 mL of anhydrous pyridine under a N₂ atmosphere and maintained at 0 °C was treated with triflic anhydride (Tf₂O; 0.06 mL; 0.32 mmol) dropwise. The resulting mixture was stirred at 0 °C for 5 minutes and at room temperature for 16 hours. (The pyridine and Tf₂O should be pure for good results. Occasionally an additional amount of Tf₂O is necessary to force the reaction to completion.) The mixture was then poured to a cold solution of citric acid and extracted with ethyl acetate to obtain an almost pure product (yield: 127 mg, about 99%). ¹H NMR (300 MHz, DMSO-d₆) δ 5.34 (s, 2H), 7.35 (m, 7H), 7.60 (m, 2H), 8.48 (s, 1H). MS (DCI/NH₃) m/z 429 (M+H)⁺, 446 (M+NH₄)⁺.

### Example 4 (reference example)

### 2-Benzyl-4-(4-fluorophenyl)-5-[4-(methylthio)phenyl]-3(2H)-pyridazinone

A mixture of the product from Example 3 (154 mg, 0.36 mmol), 4-(methylthio)benzeneboronic acid (67 mg, 0.4 mmol), Et₃N (0.11 mmol; 0.8 mmol) and Pd(Ph₃P)₄ (30 mg, 0.025 mmol) in 15 mL of toluene was heated at reflux, about 100 °C for about 45 minutes. The mixture was concentrated in vacuo and the residue purified by column chromatography (hexanes-ethyl acetate 3:1) to provide the title compound (yield: 98 mg, 68%). ¹H NMR (300 MHz, CDCl₃) δ 2.47 (s, 3H), 5.38 (s, 2H), 6.98 (m, 4H), 7.12 (m, 2H), 7.20 (m, 2H), 7.35 (m, 3H), 7.54 (m, 2H), 7.86 (s, 1H). MS (DCI/NH₃) m/z 403 (M+H)⁺, 420 (M+NH₄)⁺.

### Example 5 (reference example)

### 2-Benzyl-4-(4-fluorophenyl)-5-[4-(methylsulfonyl)phenyl]-3(2H)-pyridazinone

To a solution of the product from Example 4 (140 mg, 0.348 mmol), in 10 mL of CH₂Cl₂, at 0 °C was added peracetic acid (CH₃COOOH; 0.5 mL; 30%). The mixture was stirred at 0 °C for 90 minutes. The dichloromethane was then removed in vacuo. The residue was dissolved in ethyl acetate, washed with 10% NaHCO₃, and brine: The ethyl acetate was removed under reduced pressure. The residue was chromatographed (silica gel, CH₂Cl₂-diethyl ether 19:1) to provide the title compound (yield: 130 mg, 86%). ¹H NMR (300 MHz, CDCl₃) δ 3.04 (s, 3H), 5.40 (s, 2H), 6.95 (m, 2H), 7.16 (m, 2H), 7.33 (m, 5H), 7.55 (m, 2H), 7.86 (m, 3H). MS (DCI/NH₃) m/z 434 (M+H)⁺, 452 (M+NH₄)⁺.

### Example 6 (reference example)

### 2-(4-Fluorophenyl)-4-(2-ethyl-1-hexyloxy)-5-[4-(methylsulfonyl)phenyl]-3(2H)-pyridazinone

To a solution of 2-ethyl-1-hexanol (65 mg, 0.5 mmol) in THF (15 mL) at room temperature was added NaH (60% oil suspension) (20 mg, 0.5 mmol) and after 10 minutes 2-(4-fluorophenyl)-4-tosyloxy-5-[4-(methylsulfonyl)phenyl]-3(2H)-pyridazinone (193 mg, 0.5 mmol) was added. The resulting mixture was stirred at room temperature for the next 2 hours. The mixture was quenched with 10% citric acid and extracted with ethyl acetate. The extract was washed with water, brine, dried with MgSO₄, and purified by chromatography (silica gel, 2:1 hexanes-ethyl acetate) to provide the desired product (yield: 140 mg, 60%). mp 120-122 °C. ¹H NMR (300 MHz, DMSO-d₆) δ 0.75 (m, 6H), 1.1 (m, 6H), 1.20 (quintet, J=7 Hz, 2H), 1.44 (m, 1H), 3.27 (s, 3H), 4.30 (d, J=6 Hz, 2H), 7.37 (t, J=9 Hz, 2H), 7.65 (m, 2H), 7.89 (d, J=9 Hz, 2H), 8.06 (d, J=9 Hz, 2H), 8.18 (s, 1H). MS (APCI+) m/z 473 (M+H)⁺; (APCI-) m/z 507 (M+Cl)⁻. Anal. calc. for C₂₅H₂₉FN₂O₄S.0.5 H₂O: C, 62.35; H, 6.27; N, 5.87. Found: C, 62.22; H, 6.14; N, 6.22.

### Example 7 (reference example)

### Example 7A

### 2-(4-Fluorophenyl)-4,5-dibromo-3(2H)-pyridazinone

Mucobromic acid (5.0 g, 19.4 mmol) dissolved in acetic acid (110 mL) was treated with 4-fluorophenyl hydrazine.HCl, and the heterogeneous mixture brought to reflux at a bath temperature of 115 °C for 15 hours. During the course of reaction, the mixture became a homogeneous deep red solution, and upon cooling to 23 °C, a crystalline precipitate formed. The solution was poured into ice water (1000 mL) and stirred for 20 minutes. The yellow/brown crystals were filtered off, washed with additional cold water, and dried in vacuo to provide 5.8 g (86%) of product. (J. Het. Chem.., 1993, 30,1501; Heterocycles 1985, 23, 2603) ¹H NMR (300 MHz, DMSO-d₆) δ 7.31-7.41 (m, 2H), 7.57-7.64 (m, 2H), 8.29 (s, 1H). MS (DCI+) m/z 347 (Br₇₉Br₇₉ M+H)⁺, m/z 349 (Br₇₉Br₈₁ M+H)⁺, m/z 364 (Br₇₉Br₇₉ M+NH₄)⁺, and m/z 366 (Br₇₉Br₈₁ M+NH₄)⁺.

### Example 7B

### 2-(4-Fluorophenyl)-4-methoxy-5-bromo-3(2H)-pyridazinone

A 23 °C homogeneous solution of 2-(4-fluorophenyl)-4,5-dibromo-3(2H)-pyridazinone (7.18 g, 20.6 mmol) prepared above in tetrahydrofuran (322 mL) was treated with methanol (0.843 mL, 20.8 mmol) and after 5 minutes with NaH (0.833 g, 20.8 mmol, 60% oil dispersion). The reaction exothermed for several minutes and then was continued for 8 hours at 23 °C (Note: several reactions have run to completion at this point). The reaction did not run to completion, and so the temperature was raised to reflux for 4 hours more. The reaction was still not completed. An additional 0.1 equivalent of NaOMe solution was prepared in a separate flask as above with the quantities: 32 mL of tetrahydrofuran, 0.084 mL of methanol, and 83 mg of 60% NaH oil dispersion. This NaOMe solution was added via syringe to the reaction mixture cooled to 23 °C, and then the temperature raised to reflux for 4 hours The reaction was still not complete, and so another 0.1 equivalent NaOMe solution was prepared, added, and the reaction brought to reflux, as above. After this 4 hours, the reaction was completed. The mixture was cooled to 23 °C and diluted to 2000 mL with water. The yellow/white precipitate that formed was filtered off, washed with additional water, and concentrated in vacuo to provide 5.39 g (88%) of product. (J. Het. Chem., 1988, 25, 1757) ¹H NMR (300 MHz, DMSO-d₆) δ 4.13 (s, 3H), 7.30-7.40 (m, 2H), 7.56-7.62 (m, 2H), 8.22 (s, 1H). MS (APCI+) m/z 299 (⁷⁹Br M+H)⁺ and m/z 301 (⁸¹Br M+H)⁺.

### Example 7C

### 2-(4-Fluorophenyl)-4-methoxy-5-[4-(methylthio)phenyl]-3(2H)-pyridazinone

The title compound was prepared according to the method of Example 1 starting with 2-(4-fluorophenyl)-4-methoxy-5-bromo-3(2H)-pyridazinone in place of 2-benzyl-4-bromo-5-methoxy-3(2H)-pyridazinone and substituting 4-(methylthio)benzeneboronic acid in place of 4-fluorobenzeneboronic acid (yield: 70 mg, 61%). ¹H NMR (500 MHz, DMSO-d₆) δ 2.54 (s, 3H), 4.02 (s, 3H), 7.35 (dd, J=9.0, 9.0 Hz, 2H), 7.39 (d, J=8.5 Hz, 2H), 7.61 (d, J=8.5 Hz, 2H), 7.65 (dd, J=9.0, 5.0 Hz, 2H), 8.14 (s, 1H). MS (APCI+) m/z 343 (M+H)⁺.

### Example 8 (reference example)

### 2-(3-Chlorophenyl)-4-hydroxy-5-[4-(methylsulfonyl)phenyl]-3(2H)-pyridazinone

A suspension of 2-(3-chlorophenyl)-4-(methoxy)-5-[4-(methylsulfonyl)phenyl]-3(2H)-pyridazinone (6.26 g, 16 mmol) in 5% NaOH (54 mL) dioxane (39.4 mL) was heated at reflux and stirred for 1.5 hours. As the reaction proceeds, the solution becomes orange and homogeneous. The mixture was cooled and poured into 1N HCl, with constant stirring. The resulting white solid was filtered and rinsed with H₂O and left to dry overnight. The mostly dry product was taken up in CH₂Cl₂ and azeotroped with toluene to remove any remaining H₂O, to provide the desired product as a white solid (yield: 6.79 g, >100%). ¹H NMR (300 MHz, DMSO d₆) δ 2.27 (s, 3H), 7.51-7.62 (m, 2H), 7.68 (m, 1H), 7.79 (m, 1H), 8.03 (m, 4H), 8.24 (s, 1H). MS (DCI/NH₃) m/z 377 (M+H)⁺, 396 (M+NH₄)⁺.

### Example 9 (reference example)

### 2-(3-Chlorophenyl)-4-tosyloxy-5-[4-(methylsulfonyl)phenyl]-3(2H)-pyridazinone

To a 0 °C solution of 2-(3-chlorophenyl)-4-hydroxy-5-[4-(methylsulfonyl)phenyl]-3(2H)-pyridazinone, prepared in Example 8, (6.79 g, 16 mmol) in pyridine (160 mL) was added p-toluenesulfonyl chloride (3.06 g, 16 mmol). The solution was left to warm slowly to room temperature with stirring under nitrogen. After 2.5 hours, the mixture was poured into H₂O with constant stirring. The resulting off-white solid was filtered, rinsed with H₂O and dried to provide the desired product (yield: 6.26 g, 79%). mp 198-200 °C. ¹H NMR (300 MHz, DMSO d₆) δ 2.35 (s, 3H), 3.28 (s, 3H), 7.20 (m, 2H), 7.52-7.64 (M, 5H), 7.70 (m, 3H), 7.89 (m, 2H), 8.32 (s, 1H). MS APCI+ 531 (M+H)⁺, 548 (M+H₂O)⁺, APCI-493 (M+35)⁻. Anal. calc. for C₂₄H₁₉ClN₂O₆S₂: C, 54.29; H, 3.61; N, 5.28. Found: C, 54.55; H, 3.46; N, 5.57.

### Example 10 (reference example)

### 2-(3-Chlorophenyl)-4-(2-methylpropoxy)-5-[4-(methylsulfonyl)phenyl]-3(2H)-pyridazinone

To a stirred suspension of 2-(3-chlorophenyl)-4-tosyloxy-5-[4-(methylsulfonyl)phenyl]-3(2H)-pyridazinone, prepared in Example 9, (0.175 g, 0.33 mmol) in THF (3.3 mL) was added isobutanol (0.03 mL, 0.33 mmol), and NaH (0.0132 g, 0.33 mmol). The resulting solution was stirred under nitrogen for 1 hour. The reaction was poured into H₂O and extracted with ethyl acetate. The combined organics were dried over MgSO₄ and concentrated in vacuo. The crude solid was purified using flash chromatography (SiO₂, 2:1 hexanes:ethyl acetate) to provide the desired product (yield: 0.1088 g 76%). mp 166-169 °C. ¹H NMR (300 MHz, DMSO d₆) δ 0.78 (d, J=6 Hz, 6H), 1.84 (m, 1H), 3.29 (s, 3H), 4.20 (d, J=6 Hz, 2H), 7.51-7.63 (m, 3H), 7.76 (m, 1H), 7.92 (m, 2H), 8.07 (m, 2H), 8.21 (s, 1H). MS (DCI/NH₃) m/z 433 (M+H)⁺, 450 (M+NH₄)⁺. Anal. calc. for C₂₁H₂₁ClN₂O₄S: C, 57.07; H, 5.01; N, 6.33. Found: C, 57.06; H, 4.78; N, 6.13.

### Example 11 (reference example)

### 2-(4-Fluorophenyl)-4-(3-methylbutoxy)-5-[4-(methylsulfonyl)phenyl]-3(2H)-pyridazinone

2-(4-Fluorophenyl)-4-methoxy-5-bromo-3(2H)-pyridazinone (Example 7B) was converted into 2-(4-fluorophenyl)4-methoxy-5-[4-(methylthio)phenyl]-3(2H)-pyridazinone according to the method of Example 7C followed by the oxidation method in Example 5. The methoxy compound was converted to the 2-(4-fluorophenyl)-4-hydroxy-5-[4-(methylsulfonyl)phenyl]-3(2H)-pyridazinone, by treatment with NaOH according to the procedure of Example 8. The hydroxy compound was treated with p-toluenesulfonyl chloride according to the procedure of Example 9, to furnish 2-(4-fluorophenyl)-5-[4-(methylsulfonyl)phenyl]-4-tosyloxy-3(2H)-pyridazinone.

The title compound was prepared according to the method of Example 10, starting with 2-(4-fluorophenyl)-5-[4-(methylsulfonyl)phenyl]-4-tosyloxy-3(2H)-pyridazinone in place of 2-(3-chlorophenyl)-5-[4-(methylsulfonyl)phenyl]-4-tosyloxy-3(2H)-pyridazinone substituting 3-methyl-1-butanol in place of isobutanol (yield: 0.3932 g, 94%). mp 117-120 °C. ¹H NMR (300 MHz, DMSO d₆) δ 0.79 (d, J=6 Hz, 6H), 1.41-1.59 (m, 3H), 3.30 (s, 3H), 4.42 (d, J=5 Hz, 2H), 7.36 (m, 2H), 7.65 (m, 2H), 7.90 (m, 2H), 8.06 (m, 2H), 8.18 (s, 1H). MS (DCI/NH₃) m/z 431 (M+H)⁺, 448 (M+NH₄)⁺. Anal. calc. for C₂₂H₂₃FN₂O₄S: C, 61.38; H, 5.39; N, 6.51. Found: C, 61.42; H, 5.30; N, 6.40.

### Example 12 (reference example)

### 2-(4-Fluorophenyl)-4-(3-hydroxy-3-methyl-1-butoxy)-5-[4-(methylsulfonyl)phenyl]-3(2H)-pyridazinone

The title compound was prepared according to the method of Example 6, substituting 3-methyl-1,3-butanediol in place of 2-ethyl-1-hexanol (yield: 110 mg, 61%). mp 133-134 °C; ¹H NMR (300 MHz, DMSO-d₆) δ 1.04 (s, 6H), 1.72 (t, J = 7 Hz, 2H), 3.29 (s, 3H), 4.32 (s, 1H), 4.53 (t, J = 7 Hz, 2H), 7.37 (t, J = 9 Hz, 2H), 7.66 (m, 2H), 7.90 (d, J = 9 Hz, 2H), 8.07 (d, J = 9 Hz, 2H), 8.19 (s, 1H); MS (APCI+) m/z 447 (M+H)⁺; (APCI-) m/z 481 (M+Cl)⁻; Anal. calc. for C₂₂H₂₃FN₂O₅S·0.25 H₂O: C, 58.59; H, 5.25; N, 6.21. Found: C, 58.42; H, 5.00; N, 6.02.

### Example 13 (reference example)

### 2-(3,4-Difluorophenyl)-4-(2-hydroxy-2-methyl-1-propoxy)-5-[4-(methylsulfonyl)phenyl]-3(2H)-pyridazinone

The title compound was prepared according to the method of Example 6, substituting 2-(3,4-difluorophenyl)-4-tosyloxy-5-[4-(methylsulfonyl)phenyl]-3(2H)-pyridazinone in place of 2-(4-fluorophenyl)-4-tosyloxy-5-[4-(methylsulfonyl)phenyl]-3(2H)-pyridazinone and substituting 2-methyl-1,2-propanediol in place of 2-ethyl-1-hexanol (yield: 55 mg, 31%). ¹H NMR (300 MHz, DMSO-d₆) δ 0.97 (s, 6H), 3.30 (s, 3H), 4.20 (s, 2H), 4.54 (s, 1H), 7.52 (m, 1H), 7.62 (m, 1H), 7.81 (m, 1H), 7.98 (d, J = 9 Hz, 2H), 8.05 (d, J = 9 Hz, 2H), 8.21 (s, 1H); MS (APCI+) m/z 451 (M+H)⁺; (APCI-) m/z 485 (M+Cl)⁻; Anal. calc. for C₂₁H₂₀F₂N₂O₅S: C, 55.99; H, 4.47; N, 6.21. Found: C, 56.00; H, 4.48; N, 5.87.

### Example 14 (reference example)

### 2-(3,4-Difluorophenyl)-4-(2-hydroxy-2-methyl-1-propoxy)-5-[4-(aminosulfonyl)phenyl]-3(2H)-pyridazinone

To a solution of 2-(3,4-difluorophenyl)-4-(2-hydroxy-2-methyl-1-propoxy)-5-[4-(methylsulfonyl)phenyl]-3(2H)-pyridazinone (Example 13) (139 mg, 0.309 mmol) and dit-butylazodicarboxylate (71.2 mg, 0.309 mmol) in THF (25 mL) at -78 °C was added dropwise a 1M solution of NaHMDS (0.93 mL, 0.928 mmol) in THF. After addition the reaction was stirred another 45 min at -78 °C (or until TLC indicated a disappearance of starting material) and then was treated with 1N NaOH (20 mL). The reaction mixture was stirred at room temperature for the next 18 h. Sodium acetate trihydrate (758 mg, 5.57 mmol) was added followed by addition of hydroxylamine-O-sulphonic acid (630 mg, 5.57 mmol) and H₂O (50 mL). The resulting mixture was stirred at ambient temperature for the next 18 hours and then extracted with EtOAc. The extract was washed with water, brine, dried over MgSO₄ and concentrated in vacuo. The residue was purified by chromatography (silica gel, 1:1 hexanes-EtOAc) to provide the title compound (yield: 25 mg; 18%). mp 65-69°C; ¹H NMR (300 MHz, DMSO-d₆) δ 1.0 (s, 6H), 4.2 (s, 2H), 4.56 (s, 1H), 7.51 (m, 3H), 7.6 (m, 1H), 7.85 (m, 1H), 7.95 (s, 4H), 8.21 (s, 1H); MS (DCI/NH₃) m/z 451 (M+H)⁺, 467 (M+NH₄)⁺.

### Example 15 (reference example)

### (S)-2-(3,4-Difluorophenyl)-4-(3-hydroxy-2-methyl-1-propoxy)-5-[4-(methylsulfonyl)phenyl]-3(2H)-pyridazinone

### Example 15A

### (R)-3-t-Butoxy-2-methyl-1-propanol

A solution of (S)-(+)-methyl 3-hydroxy-2-methylpropionate (1.18 g, 10 mmol) in t-butyl acetate (30 mL) was treated with 70% HClO₄ (0.1 mL) and the reaction mixture was left at room temperature in a tightly closed flask for 24 hours. The mixture was then poured into a saturated solution of NaHCO₃ and extracted with ethyl ether. The ether was removed in vacuo and the residue was dissolved in THF (50 mL). To the resulting solution was added NaBH₄ (925 mg, 25 mmol) and at 55 °C dropwise methanol (10 mL). The reaction was continued at 55 °C for 1 hours, then it was cooled to ambient temperature, acidified with 10% citric acid to pH 5 and extracted with ethyl acetate. The acetate extract was washed with water, brine, dried with MgSO₄ and concentrated in vacuo. The residue was chromatographed (silica gel, 2:1 hexane-ethyl acetate) to provide (R)-3-t-butoxy-2-methyl-1-propanol (yield: 1 g, 68%). ¹H NMR (300 MHz, CDCl₃) δ 0.85 (d, J = 7 Hz, 3H), 1.20 (s, 9H), 2.03 (m, 1H), 3.30 (t, J = 12 Hz, 1H), 3.53 (dd, J = 4.5 Hz, 12 Hz, 1H), 3.70 (m, 2 H); MS (DCI/NH₃) m/z 164 (M+NH₄)⁺.

### Example 15B

### (S)-2-(3,4-Difluorophenyl)-4-(3-t-butoxy-2-methyl-1-propoxy)-5-(4-methylsulfonyl)phenyl]-3(2H)-pyridazinone

To a solution 2-(3,4-difluorophenyl)-4-hydroxy-5-[4-(methylsulfonyl)phenyl-3(2H)-pyridazinone (378 mg, 1 mmol), Ph₃P (524 mg, 2 mmol) and Example 15A (146 mg, 1 mmol) in THF (25 mL) at room temperature was added dropwise a solution of DIAD (0.4 mL, 2 mmol) in THF (5 mL). The mixture was then stirred at room temperature for 6 hours and concentrated in vacuo. The residue was passed through a silica gel pad (hexane-ethyl acetate as an eluent) to provide 550 mg of roughly purified (S)-2-(3,4-difluorophenyl)-4-(3-t-butoxy-2-methyl-1-propoxy)-5-[4-(methylsulfonyl)phenyl]-3(2H)-pyridazinone, still contaminated with reduced DIAD. MS (APCI+) m/z 507 (M+H)⁺; (APCI-) m/z 541 (M+Cl)⁻.

### Example 15C

### (S)-2-(3,4-Difluorophenyl)-4-(3-hydroxy-2-methyl-1-propoxy)-5-[4-(methylsulfonyl)phenyl]-3(2H)-pyridazinone

A mixture of Example 15B (100 mg, ~0.2 mmol) in TFA (5 mL) was left at ambient temperature for 24 hours. The mixture was then concentrated in vacuo, the residue was neutralized with saturated solution of NaHCO₃ and extracted with ethyl acetate. Purification on a column (silica gel, 1:2 hexanes-EtOAc) gave a foamy product (yield: 51 mg, 56%). ¹H NMR (300 MHz, DMSO-d₆) δ 0.75 (d, J = 7 Hz, 3H), 1.81 (sextet (J = 7 Hz, 1H), 3.21 (d, J = 6 Hz, 2H), 3.30 (s, 3H), 4.29 (dd, J = 6 Hz, 12 Hz, 1H), 4.40 (dd, J = 6 Hz, 12 Hz, 1H), 4.48 (br s, 1H), 7.52 (m, 1H), 7.61 (m, 1H), 7.80 (m, 1H), 7.91 (d, J = 9 Hz, 2H), 8.07 (d, J = 9 Hz, 2H), 8.20 (s, 1H); MS (APCI+) m/z 451 (M+H)⁺; (APCI-) m/z 485 (M+Cl)-; Anal. calc. for C₂₁H₂₀F₂N₂O₅S: C, 55.99; H, 4.47; N, 6.21. Found: C, 55.65; H, 4.65; N, 5.92.

### Example 16 (reference example)

### (R)-2-(3,4-Difluorophenyl)-4-(3-hydroxy-2-methyl-1-propoxy)-5-[4-(methylsulfonyl)phenyl]-3(2H)-pyridazinone

The title compound was prepared by the method described in Example 15, substituting (R)-(-)-methyl 3-hydroxy-2-methylpropionate in place of (S)-(+)-methyl 3-hydroxy-2-methylpropionate (yield: 65 mg, 61%). ¹H NMR (300 MHz, DMSO-d₆) δ 0.75 (d, J = 7 Hz, 3H), 1.81 (sextet, J = 7 Hz, 1H), 3.21 (t, J = 6 Hz, 2H), 3.30 (s, 3H), 4.29 (dd, J = 6 Hz and 12 Hz, 1H), 4.40 (dd, J = 6 Hz, 12 Hz, 1H), 4.49 (t, J = 6 Hz, 1H), 7.52 (m, 1H), 7.61 (m, 1H), 7.80 (m, 1H), 7.91 (d, J = 9 Hz, 2H), 8.07 (d, J = 9 Hz, 2H), 8.20 (s, 1H); MS (APCI+) m/z 451 (M+H)⁺; (APCI-), m/z 485 (M+Cl)-. Anal. calc. for C₂₁H₂₀F₂N₂O₅S: C, 55.99; H, 4.47; N, 6.21. Found: C, 55.62; H, 4.52; N, 6.06.

### Example 17 (reference example)

### (S)-2-(3,4-Difluorophenyl)-4-(3-hydroxy-2-methyl-1-propoxy)-5-[4-(aminosulfonyl)phenyl]-3(2H)-pyridazinone

To a solution of (S)-2-(3,4-difluorophenyl)-4-(3-t-butoxy-2-methyl-1-propoxy)-5-(4-(methylsulfonyl)phenyl]-3(2H)-pyridazinone (Example 15B, 450 mg, ~0.9 mmol) and DBAD (207 mg, 0.9 mmol) in THF (25 mL) at -78 °C was added dropwise a1M solution of LiHMDS (3 mL, 3 mmol) and the resulting mixture was stirred at -78 °C for 2 hours. The mixture was warmed to room temperature and 1N NaOH (5 mL, 5 mmol) was added. After 12 hours at ambient temperature, sodium acetate trihydrate (2.76 g, 20 mmol) and water (10 mL) followed by hydroxylamine-O-sulfonic acid (2g, 15 mmol) were added and the mixture was stirred at room temperature for 5 hours. The product was extracted with ethyl acetate and purified by chromatography (silica gel, 1:2 hexanes-EtOAc) to afford crude intermediate (yield: 160 mg, 35%). MS (APCI+) m/z 508 (M+H)⁺; (APCI-) m/z 542 (M+Cl)-.

TFA (5 mL) was added to the above intermediate and the resulting solution was left at room temperature for 24 hours. The TFA was removed in vacuo, then the residue was neutralized with saturated NaHCO₃ and extracted with ethyl acetate. The organic layer was dried over MgSO₄ then filtered. The filtrate was concentrated in vacuo and the residue was purified by column chromatography (silica gel, 1:2 hexane-ethyl acetate) to provide the title compound (yield: 50 mg, 33%). ¹H NMR (300 MHz, DMSO-d₆) δ 0.76 (d, J = 7 Hz, 3H), 1.81 (sextet, J = 7 Hz, 1H), 3.22 (t, J = 6 Hz, 2H), 4.28 (dd, J = 6 Hz, 12 Hz, 1H), 4.40 (dd, J = 6 Hz, 12 Hz, 1H), 4.50 (t, J = 6 Hz, 1H), 7.51 (m, 3H), 7.61 (m, 1H), 7.80 (m, 1H), 7.84 (d, J = 9 Hz, 2H), 7.95 (d, J = 9 Hz, 2H), 8.20 (s, 1H); MS (APCI+) m/z 452 (M+H)⁺; (APCI-) m/z 486 (M+Cl)-.

### Example 18 (reference example)

### (R)-2-(3,4-Difluorophenyl)-4-(3-hydroxy-2-methyl-1-propoxy)-5-[4-(aminosulfonyl)phenyl]-3(2H)-pyridazinone

The desired material was prepared according to the procedure of Example 17 substituting (R)-2-(3,4-difluorophenyl)-4-(3-t-butoxy-2-methyl-1-propoxy)-5-[4-(methylsulfonyl)phenyl]-3(2H)-pyridazinone in place of (S)-2-(3,4-difluorophenyl)-4-(3-t-butoxy-2-methyl-1-propoxy)-5-[4-(methylsulfonyl)phenyl]-3(2H)-pyridazinone (yield: 30 mg, 20%). ¹H NMR (300 MHz, DMSO-d₆) δ 0.76 (d, J = 7 Hz, 3H), 1.81 (sextet, J = 7 Hz, 1H), 3.22 (t, J = 6 Hz, 2H), 4.28 (dd, J = 6 Hz, 12 Hz, 1H), 4.40 (dd, J = 6 Hz and 12 Hz, 1H), 4.50 (t, J = 6 Hz, 1H), 7.51 (m, 3H), 7.61 (m, 1H), 7.80 (m, 1H), 7.84 (d, J = 9 Hz, 2H), 7.95 (d, J = 9 Hz, 2H), 8.20 (s, 1H); MS (APCI+) m/z 452 (M+H)⁺; (APCI-) m/z 486 (M+Cl)-. Anal. calc. for C₂₀H₁₉F₂N₃O₅S: C, 53.21; H, 4.24; N, 9.30. Found: C, 53.45; H, 5.53; N, 9.50.

### Example 19 (reference example)

### 2-(4-Fluorophenyl)-4-(4-hydroxy-3-methyl-1-butoxy)-5-[4-(methylsulfonyl)phenyl]-3(2H)-pyridazinone

The title compound was prepared according to the method of Example 6, substituting 2-methyl-1,4-butandiol in place of 2-ethyl-1-hexanol and separating the regioisomeric products by multiple preparative thin layer chromatography runs, eluting with 4:1 ethyl acetate/hexanes (yield: 65 mg, 19%). ¹H NMR (300 MHz, CDCl₃) δ 0.87 (d, J = 8.1 Hz, 3H), 1.48-1.87 (m, 4H), 3.13 (s, 3H), 3.41 (dd, J = 6.3, 13.5 Hz, 1H), 3.46 (dd, J = 6.3, 13.5 Hz, 1H), 4.48-4.63 (m, 2H), 7.15-7.24 (m, 2H), 7.58-7.66 (m, 2H), 7.79 (d, J = 10.5 Hz, 2H), 7.91 (s, 1H), 8.07 (d, J = 10.5 Hz, 2H); MS (APCI+) m/z 447 (M+H)⁺.

### Example 20

### 2-(4-Fluorophenyl)-4-(4-hydroxy-2-methyl-1-butoxy)-5-[4-(methylsulfonyl)phenyl]-3(2H)-pyridazinone

The title compound was prepared according to the method of Example 6, substituting 2-methyl-1,4-butandiol in place of 2-ethyl-1-hexanol and separating the regioisomeric products by multiple preparative thin layer chromatography runs, eluting with 4:1 ethyl acetate/hexanes (yield: 43 mg, 12%). ¹H NMR (300 MHz, CDCl₃) δ 0.87 (d, J = 8.1 Hz, 3H), 1,33-1.46 (m, 1H), 1.50-1.67 (m, 2H), 1.93-2.04 (m, 1H), 3.13 (s, 3H), 3.54-3.72 (m, 2H), 4.29 (dd, J = 6.0, 9.3 Hz, 1H), 4.43 (dd, J = 6.0, 9.3 Hz, 1H), 7.15-7.24 (m, 2H), 7.58-7.66 (m, 2H), 7.79 (d, J = 10.5 Hz, 2H), 7.91 (s, 1H), 8.07 (d, J = 10.5 Hz, 2H); MS (APCI+) m/z 447 (M+H)⁺.

### Example 21

### 2-(4-Fluorophenyl)-4-(3-hydroxy-3-methyl-1-butoxy)-5-[4-(aminosulfonyl)phenyl]-3(2H)-pyridazinone

The title compound was prepared according to the method of Example 14, substituting 2-(4-fluorophenyl)-4-(3-hydroxy-3-methyl-1-butoxy)-5-[4-(methylsulfonyl)phenyl]-3(2H)-pyridazinone (Example 12) in place of 2-(3,4-difluorophenyl)-4-(2-hydroxy-2-methyl-1-propoxy)-5-[4-(methylsulfonyl)phenyl]-3(2H)-pyridazinone (yield: 600 mg, 60%). mp 163-164°C; ¹H NMR (300 MHz, DMSO-d₆) δ 1.05 (s, 6H), 1.73 (t, J = 6 Hz, 2H), 4.30 (s, 1H), 4.52 (t, J = 6 Hz, 2H), 7.37 (t, J = 9 Hz, 1H), 7.47 (s, 2H), 7.65 (dd, J = 9 Hz, J = 3 Hz, 2H), 8.83 (d, J = 9 Hz, 2H), 8.95 (d, J = 9 Hz, 2H), 8.18 (s, 1H); MS (DCI/NH₃) m/z 448 (M+H)⁺. Anal. calcd. for C₂₁H₂₂FN₃O₅S: C, 56.36; H, 4.95; N, 9.39. Found: C, 55.96; H, 4.89; N, 9.09.

### Example 22

### 2-(3,4-Difluorophenyl)-4-(3-hydroxy-3-methyl-1-butoxy)-5-[4-(methylsulfonyl)phenyl]-3(2H)-pyridazinone

### Example 22A

### 3,4-Difluorophenylhydrazine

A stirred mixture of 3,4-difluoroaniline (12.9 g, 0.1 mol) in concentrated hydrochloric acid (60 mL) was chilled to -10°C with an ice/methanol bath. A solution of sodium nitrite (6.9 g, 0.1 mol) in water (30 mL) was added at a rate which maintained the temperature of the reaction mixture below 10°C. After stirring for 2 hours, the reaction mixture was cooled to 0°C and a solution of tin(II) chloride (56.88 g, 0.3 mol) in concentrated hydrochloric acid (50 mL) was added dropwise with vigorous stirring. Additional concentrated hydrochloric acid (150 mL) was added to the reaction mixture and stirring was continued for 2 hours. The reaction mixture was filtered to collect the precipitated hydrochloride salt of the title compound. This precipitate was dissolved in water (75 mL) and the resulting solution was basified with 50% aqueous sodium hydroxide and extracted with ethyl acetate. The organic extracts were dried (Na₂SO₄) and filtered. The filtrate was concentrated in vacuo to provide the title intermediate as a brown oil (8.11 g, 57.4%).

### Example 22B

### 2-(3,4-Difluorophenyl)-4,5-dibromo-3(2H)-pyridazinone

The title intermediate was prepared by the method of Example 7A, substituting 3,4-difluorophenylhydrazine (Example 22A) for 4-fluorophenylhydrazine hydrochloride.

### Example 22C

### 2-(3,4-Difluorophenyl)-4-(3-hydroxy-3-methyl-1-butoxy)-5-bromo-3(2H)-pyridazinone

The dibromo-intermediate from Example 22B was reacted according to the procedure described in Example 7B, substituting 3-methyl-1,3-butanediol in place of methanol, to selectively react at the 4-position and provide the title intermediate.

### Example 22D

### 2-(3,4-Difluorophenyl)-4-(3-hydroxy-3-methyl-1-butoxy)-5-[4-(methylthio)phenyl]-3(2H)-pyridazinone

The product from Example 22C (12.79 g, 32.86 mmol) was coupled to 4-(methylthio)phenylboronic acid (6.07 g, 36.15 mmol) with K₂CO₃ (10 g, 72.3 mmol) and PdCl₂(PPh₃)₂ (1.15 g, 1.64 mmol) in ethanol (200 mL) at 60-65 °C for 40-70 minutes to provide the title intermediate (yield: 9.16 g, 64.5%).

### Example 22E

### 2-(3,4-Difluorophenyl)-4-(3-hydroxy-3-methyl-1-butoxy)-5-[4-(methylsulfonyl)phenyl]-3(2H)-pyridazinone

The sulfide from Example 22D was oxidized to the title compound by the method of Example5 (yield: 7.46 g, 76%). m.p. 131-133°C; ¹H NMR (300 MHz, DMSO-d₆) δ 1.04 (s, 6H), 1.73 (t, J = 6 Hz, 2H), 3.29 (s, 3H), 4.43 (s, 1H), 4.54 (t, J = 6 Hz, 2H), 7.52 (m, 1H), 7.62 (ddd, J = 9 Hz, J = 9 Hz, J = 1.5 Hz, 1H), 7.82 (ddd, J = 9 Hz, J = 9 Hz, J = 3Hz, 1H), 7.91 (d, J = 9 Hz, 1H), 8.08 (d, J = 9 Hz, 2H), 8.20 (s, 1H); MS (DCI-NH₃) m/e 465 (M+H)⁺. Anal. calcd. for C₂₂H₂₂F₂N₂O₅S: C, 56.88; H, 4.77; N, 6.03. Found: C, 56.92; H, 4.88; N, 5.94.

### Example 23

### 2-(3,4-Difluorophenyl)-4-(3-hydroxy-3-methyl-1-butoxy)-5-[4-(aminosulfonyl)phenyl]-3(2H)-pyridazinone

The title compound was prepared according to the method of Example 14, substituting 2-(3,4-difluorophenyl)-4-(3-hydroxy-3-methyl-1-butoxy)-5-[4-(methylsulfonyl)phenyl]-3(2H)-pyridazinone (Example 22) in place of 2-(3,4-difluorophenyl)-4-(2-hydroxy-2-methyl-1-propoxy)-5-[4-(methylsulfonyl)phenyl]-3(2H)-pyridazinone (yield: 300 mg, 50%). mp 181-181°C; ¹H NMR (300 MHz, DMSO-d₆) δ 1.04 (s, 6H), 1.72 (t, J = 6 Hz, 2H), 4.43 (s, 1H), 4.53 (t, J = 6 Hz, 2H), 7.49 (s, 2H), 7.53 (m, 1H), 7.63 (ddd, J = 9 Hz, J = 9 Hz, J = 1.5 Hz, 1H), 7.79 (ddd, J = 9 Hz, J = 9 Hz, J = 3 Hz, 1H), 7.83 (d, J = 9 Hz, 1H), 7.95 (d, J = 9 Hz, 2H), 8.19 (s, 1H); MS (DCI/NH₃) m/z 466 (M+H)⁺. Anal. calcd. for C₂₁H_{21F2}N₂O₅S: C, 54.12; H, 4.66; N, 8.81. Found: C, 54.19; H, 4.55; N, 9.03.

### Example 24

### 2-(3-Chloro-4-fluorophenyl)-4-(3-hydroxy-3-methyl-1-butoxy)-5-[4-(methylsulfonyl)phenyl]-3(2H)-pyridazinone

The title compound was prepared according to the sequence of reactions described in Example 22, substituting 3-chloro-4-fluorophenylhydrazine in place of 3,4-difluorophenylhydrazine (yield: 200 mg, 89%). mp 108-110°C; ¹H NMR (300 MHz, CDCl₃) δ 1.24 (s, 6H), 1.89 (t, 2H, J = 6 Hz), 2.34 (s, 1H), 3.12 (s, 3H), 4.57 (t, J = 6 Hz, 2H), 7.25 (t, J = 9 Hz, 1H), 7.60 (m, 1H), 7.78 (d, J = 6 Hz, 1H), 7.79 (d, J = 9 Hz, 2H), 7.92 (s, 1H), 8.08 (d, J = 9 Hz, 2H); MS (DCI/NH₃) m/z 481 (M+H)⁺; Anal. calcd. for C₂₂H₂₂FClN₂O₅S: C, 54.94; H, 4.61; N, 5.82. Found: C, 54.87; H, 4.65; N, 5.72.

### Example 25

### 2-(3-Chloro-4-fluorophenyl)-4-(3-hydroxy-3-methyl-1-butoxy)-5-[4-(aminosulfonyl)phenyl]-3(2H)-pyridazinone

The title compound was prepared according to the method of Example 14, substituting 2-(3-chloro-4-fluorophenyl)-4-(3-hydroxy-3-methyl-1-butoxy)-5-[4-(methylsulfonyl)phenyl]-3(2H)-pyridazinone (Example 24) in place of 2-(3,4-difluorophenyl)-4-(2-hydroxy-2-methyl-1-propoxy)-5-[4-(methylsulfonyl)phenyl]-3(2H)-pyridazinone (yield: 160 mg, 45%). mp 59-62°C; ¹H NMR (300 MHz, DMSO-d₆) δ 1.05 (s, 6H), 1.73 (t, 2H, J = 6 Hz), 4.32 (s, 1H), 4.54 (t, J = 6 Hz, 2H), 7.50 (s, 2H), 7.60 (t, J = 9 Hz, 1H), 7.66 (m, 1H), 7.73 (d, J = 9 Hz, 2H), 7.74 (d, J = 9 Hz, 2H), 7.75 (m, 1H), 8.22 (s, 1H); MS (DCI/NH₃) m/z 482 (M+H)⁺. Anal. calcd. for C₂₁H₂₁FClN₃O₅S: C, 52.33; H, 4.39; N, 8.71. Found: C, 52.30; H, 5.03; N, 8.10.

### Example 26

### 2-(3-Chlorophenyl)-4-(3-hydroxy-3-methyl-1-butoxy)-5-[4-(methylsulfonyl)phenyl]-3(2H)-pyridazinone

The title compound was prepared according to the sequence of reactions described in Example 22, substituting 3-chlorophenylhydrazine in place of 3,4-difluorophenylhydrazine (yield: 200 mg, 89%). ¹H NMR (300 MHz, CDCl₃) δ 8.04 (d, J = 8.5 Hz, 2H), 7.91 (s, 1H), 7.77 (d, J = 8.5 Hz, 2H), 7.67 (m, 1H), 7.57 (m, 1H), 7.40 (t, J = 8.8 Hz, 1H), 7.36 (m, 1H), 4.54 (t, J = 6.3 Hz, 2H), 3.10 (s, 3H), 2.56 (s, 1H), 1.86 (t, J = 6.3 Hz, 2H), 1.20 (s, 6H), MS (DCI/NH₃) m/z 462(M+H)⁺.

### Example 27

### 2-(3-Chlorophenyl)-4-(3-hydroxy-3-methyl-1-butoxy)-5-[4-(aminosulfonyl)phenyl]-3(2H)-pyridazinone

The title compound was prepared according to the method of Example 14, substituting 2-(3-chlorophenyl)-4-(3-hydroxy-3-methyl-1-butoxy)-5-[4-(methylsulfonyl)phenyl]-3(2H)-pyridazinone (Example 26) in place of 2-(3,4-difluorophenyl)-4-(2-hydroxy-2-methyl-1-propoxy)-5-[4-(methylsulfonyl)phenyl]-3(2H)-pyridazinone. ¹H NMR (300 MHz, CDCl₃) δ 8.03 (d, J = 8.7 Hz, 2H), 7.91 (s, 1H), 7.70 (d, J = 8.7 Hz, 2H), 7.68 (m, 1H), 7.57 (m, 1H), 7.41 (m, 1H), 7.38 (m, 1H), 5.65 (s, 2H), 4.51(t, J = 6.6 Hz, 2H), 2.70 (br s, 1H), 1.87 (t, J = 6.6 Hz, 2H), 1.20 (s, 6H); MS (DCI/NH₃) m/z 463 (M+H)⁺.

### Example 28

### 2-(4-Fluorophenyl)-4-(2-hydroxy-2-methyl-1-propoxy)-5-[4-(methylsulfonyl)phenyl]-3(2H)-pyridazinone

The title compound was prepared according to the sequence of reactions described in Example 22, substituting 4-fluorophenylhydrazine in place of 3,4-difluorophenylhydrazine and substituting 2-methyl-1,2-propanediol (prepared by the LAH reduction of methyl 2-hydroxyisobutyrate) in place of 3-methyl-1,3-butanediol. mp 152-154°C, ¹H NMR (300 MHz, CDCl₃) δ 8.10 (d, 2H, J = 18 Hz), 7.95 (s, 1H), 7.83 (d, 2H, J = 18 Hz), 7.63 (d, 1H, J = 18 Hz), 7.61 (d, 1H, J = 18 Hz), 4.18 (s, 2H), 3.13 (s, 3H), 1.19 (s, 6H), MS (DCI/NH₃) m/z 433 (M+H)⁺, 450 (M+NH₄)⁺, Analysis for C₂₁H₂₁FN₂O₅S, Calcd: C, 58.32; H, 4.89; N, 6.48. Found: C, 58.42; H, 5.05; N, 6.43.

### Example 29

### 2-(4-Fluorophenyl)-4-(2-hydroxy-2-methyl-1-propoxy)-5-[4-(aminosulfonyl)phenyl]-3(2H)-pyridazinone

The title compound was prepared according to the method of Example 14, substituting 2-(4-fluorophenyl)-4-(2-hydroxy-2-methyl-1-propoxy)-5-[4-(methylsulfonyl)phenyl]-3(2H)-pyridazinone (Example 28) in place of 2-(3,4-difluoro-phenyl)-4-(2-hydroxy-2-methyl-1-propoxy)-5-[4-(methylsulfonyl)phenyl]-3(2H)-pyridazinone. mp 155-158°C; ¹H NMR (300 MHz, DMSO-d₆) δ 8.17 (s, 1H), 7.92 (s, 4H), 7.67 (d, 1H, J = 18 Hz), 7.64 (d, 1H, J = 18 Hz), 7.49 (s, 2H), 7.38 (d, 1H, J = 18 Hz), 7.35 (d, 1H, J = 18 Hz), 4.54 (s, 1H), 4.19 (s, 2H), 1.00 (s, 6H), MS (ESI+): m/z 434 (M+H)⁺, 456 (M+Na)⁺, 889 (2M+Na)⁺; Analysis for C₂₀H₂₀FN₃O₅S, Calcd: C, 55.42; H, 4.65; N, 9.69. Found: C, 55.64; H, 4.85; N, 9.53.

### Example 30

### 2-(3-Chloro-4-fluorophenyl)-4-(2-hydroxy-2-methyl-1-propoxy)-5-[4-(methylsulfonyl)phenyl]-3(2H)-pyridazinone

The title compound was prepared according to the sequence of reactions described in Example 22, substituting 3-chloro-4-fluorophenylhydrazine in place of 3,4-difluorophenylhydrazine and substituting 2-methyl-1,2-propanediol (prepared by the LAH reduction of methyl 2-hydroxyisobutyrate) in place of 3-methyl-1,3-butanediol. mp 122-124 °C, ¹H NMR (300 MHz, CDCl₃) δ 0.98 (s, 6H), 3.29 (s, 3H), 4.21 (s, 2H), 4.56 (s, 1H), 7.61 (dd, 1H, J = 7,17 Hz), 7.67 (ddd, 1H, J = 2,4,7 Hz), 7.93 (dd, 1H, J = 2,7 Hz), 7.98 (d, 2H, J = 8 Hz), 8.06 (d, 2H, J = 8 Hz), 8.22 (s, 1H); MS (DCI/NH₃) m/z 465(M-H)⁻; Anal. Calcd for C₂₁H₂₀ClFN₂O₅S: C 54.02, H 4.32, N 6.00. Found: C 54.06, H4.57, N 5.95.

### Example 31

### 2-(3-Chloro-4-fluorophenyl)-4-(2-hydroxy-2-methyl-1-propoxy)-5-[4-(aminosulfonyl)phenyl]-3(2H)-pyridazinone

The title compound was prepared according to the method of Example 14, substituting 2-(3-chloro-4-fluorophenyl)-4-(2-hydroxy-2-methyl-1-propoxy)-5-[4-(methylsulfonyl)phenyl]-3(2H)-pyridazinone (Example 30) in place of 2-(3,4-difluorophenyl)-4-(2-hydroxy-2-methyl-1-propoxy)-5-[4-(methylsulfonyl)phenyl]-3(2H)-pyridazinone. mp 176-178 °C, ¹H NMR (300 MHz, CDCl₃) δ 1.00 (s, 6H), 4.19 (s, 2H), 4.54 (s, 1H), 7.49 (s, 2H), 7.62 (t, 1H, J = 9 Hz), 7.66 (ddd, 1H, J = 2,5,9 Hz), 7.92 (s, 4.5H), 7.94 (d, 0.5H, J = 2 Hz), 8.20 (s, 1H); MS (BCI/NH₃) m/z 468 (M+H)⁺; 1 Cl, 490 (M+Na)⁺;1Cl; Anal. Calcd for C₂₀H₁₉ClFN₂O₅S: C 51.34, H 4.09, N 8.98. Found: C 51.33, H 4.23, N 8.76.

### Example 32

### 2-(3-Chlorophenyl)-4-(2-hydroxy-2-methyl-1-propoxy)-5-[4-(methylsulfonyl)phenyl]-3(2H)-pyridazinone

The title compound was prepared according to the sequence of reactions described in Example 22, substituting 3-chlorophenylhydrazine in place of 3,4-difluorophenylhydrazine and substituting 2-methyl-1,2-propanediol (prepared by the LAH reduction of methyl 2-hydroxyisobutyrate) in place of 3-methyl-1,3-butanediol. ¹H NMR (300 MHz, CDCl₃) δ 8.15 (m, 2H), 7.98 (s, 1H), 7.85 (m, 2H), 7.76 (m, 1H), 7.62 (m, 1H), 7.43 (m, 2H), 4.22 (s, 2H), 3.15 (s, 3H), 1.21 (s, 6H); MS (DCI/NH₃) m/z 449 (M+H)⁺; Anal. Calcd for C₂₁H₂₁ClN₂O₅S: C 56.18, H 4.72, N 6.24. Found: C 56.08, H 4.67, N 6.23.

### Example 33

### 2-(3-Chlorophenyl)-4-(2-hydroxy-2-methyl-1-propoxy)-5-[4-(aminosulfonyl)phenyl]-3(2H)-pyridazinone

The title compound was prepared according to the method of Example 14, substituting 2-(3-chlorophenyl)-4-(2-hydroxy-2-methyl-1-propoxy)-5-[4-(methylsulfonyl)phenyl]-3(2H)-pyridazinone (Example 32) in place of 2-(3,4-difluorophenyl)-4-(2-hydroxy-2-methyl-1-propoxy)-5-[4-(methylsulfonyl)phenyl]-3(2H)-pyridazinone. ¹H NMR (400 MHz, DMSO-d₆) δ 8.19 (s, 1H), 7.93 (m, 4H), 7.75 (m, 1H), 7.61-7.48 (m, 5H), 4.53 (s, 2H), 4.20 (s, 3H), 1.00 (s, 6H); MS (ESI-) m/z 448 (M-H)⁻; Anal. Calcd for C₂₀H₂₀ClN₃O₅S: C 53.39, H 4.48, N 9.34. Found: C 53.11, H 4.82, N 9.24.

### Example 34

### 2-(2,2,2-Trifluoroethyl)-4-(2-hydroxy-2-methyl-1-propoxy)-5-[4-(methylsulfonyl)phenyl]-3(2H)-pyridazinone

The title compound was prepared according to the sequence of reactions described in Example 22, substituting 2,2,2-trifluoroethylhydrazine in place of 3,4-difluorophenylhydrazine and substituting 2-methyl-1,2-propanediol (prepared by the LAH reduction of methyl 2-hydroxyisobutyrate) in place of 3-methyl-1,3-butanediol. ¹H NMR (300MHz, CDCl₃) δ 1.18 (s, 6H), 2.62 (br s, 1H), 3.15 (s, 3H), 4.20 (s, 2H), 4.85 (q, J = 9 Hz, 2H), 7.78 (d, J = 9 Hz, 2H), 7.85 (s, 1H), 8.08 (d, J = 9 Hz, 2H); MS (DCI/NH₃) m/z 421 (M+1)⁺; Analysis calculated for C₁₇H₁₉F₃N₂O₅S: C, 48.57; H, 4.56; N, 6.66. Found: C, 48.72; H, 4.78; N, 6.56.

### Example 35

### 2-(2,2,2-Trifluoroethyl)-4-(2-hydroxy-2-methyl-1-propoxy)-5-[4-(aminosulfonyl)phenyl]-3(2H)-pyridazinone

The title compound was prepared by the following sequence of reactions. Mucobromic acid and 2,2,2-trifluoroethylhydrazine hydrochloride were reacted to provide 2-(2,2,2-trifluoroethyl)-4,5-dibromo-3(2H)-pyridazinone following the procedure in Example 7A. The dibromo-intermediate was reacted according to the procedure described in Example 7B, substituting 2-methyl-1,2-propanediol in place of methanol, to selectively react at the 4-position and provide 2-(2,2,2-trifluoroethyl)-4-(2-hydroxy-2-methyl-1-propoxy)-5-bromo-3(2H)-pyridazinone. This 5-bromo-compound was coupled to 4-[2-(tetrahydropyranyl)thio]phenylboronic acid (prepared from THP-protected 4-bromothiophenol and triisopropyl borate) with K₂CO₃ and PdCl₂(PPh₃)₂ in ethanol at 60-65 °C for 40-70 minutes to provide 2-(2,2,2-trifluoroethyl)-4-(2-hydroxy-2-methyl-1-propoxy)-5-[4-[2-(tetrahydropyranyl)thio]phenyl]-3(2H)-pyridazinone. This intermediate THP-sulfide was then converted to the title compound by treatment with N-chlorosuccinimide (3.5 equivalents) at 0 °C in THF/H₂O for 15 minutes to an hour followed by addition of excess ammonium hydroxide at 0 °C and stirring at ambient temperature for 3 hours. Aqueous work-up and column chromatographic purification (80:20 pentane/ethyl acetate) provided the title compound. ¹H NMR (300MHz, CDCl₃) δ 1.18 (s, 6H), 2.65 (br s, 1H), 4.15 (s, 2H), 4.85 (q, J = 9 Hz, 2H), 4.9 (s, 2H), 7.75 (d, J = 9 Hz, 2H), 7.85 (s, 1H), 8.05 (d, J = 9 Hz, 2H); MS (DCI/NH₃) m/z 422 (M+H)⁺; Analysis calculated for C₁₆H₁₈F₃N₃O₅S: C, 45.60; H, 4.30; N, 9.97. Found: C, 45.86; H, 4.63; N, 9.81.

### Example 36

### 2-(2,2,2-Trifluoroethyl)-4-(3-hydroxy-2,2-dimethyl-1-propoxy)-5-[4-(methylsulfonyl)phenyl]-3(2H)-pyridazinone

The title compound may be prepared according to the method of Example 34, substituting neopentyl glycol in place of 2-methyl-1,2-propanediol.

### Example 37

### 2-(2,2,2-Trifluoroethyl)-4-(3-hydroxy-2,2-dimethyl-1-propoxy)-5-[4-(aminosulfonyl)phenyl]-3(2H)-pyridazinone

The title compound may be prepared according to the method of Example 35, substituting neopentyl glycol in place of 2-methyl-1,2-propanediol.

### Example 38

### 2-(4-Fluorophenyl)-4-(4-hydroxy-3-methyl-1-butoxy)-5-[4-(aminosulfonyl)phenyl]-3(2H)-pyridazinone

The title compound may be prepared according to the method of Example 14, substituting 2-(4-fluorophenyl)-4-(4-hydroxy-3-methyl-1-butoxy)-5-[4-(methylsulfonyl)phenyl]-3(2H)-pyridazinone (Example 19) for 2-(3,4-difluorophenyl)-4-(2-hydroxy-2-methyl-1-propoxy)-5-[4-(methylsulfonyl)phenyl]-3(2H)-pyridazinone.

### Example 39

### 2-(3,4-Difluorophenyl)-4-(4-hydroxy-3-methyl-1-butoxy)-5-[4-(methylsulfonyl)phenyl]-3(2H)-pyridazinone

The title compound may be prepared according to the sequence of reactions described in Example 22, substituting 2-methyl-1,4-butanediol for 3-methyl-1,3-butanediol, then separating the regioisomeric products by multiple preparative thin layer chromatography runs.

### Example 40

### 2-(3-Chloro-4-fluorophenyl)-4-(4-hydroxy-3-methyl-1-butoxy)-5-[4-(methylsulfonyl)phenyl]-3(2H)-pyridazinone

The title compound may be prepared according to the sequence of reactions described in Example 22, substituting 3-chloro-4-fluorophenylhydrazine for 3,4-difluorophenylhydrazine and substituting 2-methyl-1,4-butanediol for 3-methyl-1,3-butanediol, then separating the regioisomeric products by multiple preparative thin layer chromatography runs.

### Example 41

### 2-(3-Chlorophenyl)-4-(4-hydroxy-3-methyl-1-butoxy)-5-[4-(methylsulfonyl)phenyl]-3(2H)-pyridazinone

The title compound may be prepared according to the sequence of reactions described in Example 22, substituting 3-chlorophenylhydrazine for 3,4-difluorophenylhydrazine and substituting 2-methyl-1,4-butanediol for 3-methyl-1,3-butanediol, then separating the regioisomeric products by multiple preparative thin layer chromatography runs.

### Example 42

### 2-(3,4-Difluorophenyl)-4-(4-hydroxy-3-methyl-1-butoxy)-5-[4-(aminosulfonyl)phenyl]-3(2H)-pyridazinone

The title compound may be prepared according to the method of Example 14, substituting 2-(3,4-difluorophenyl)-4-(4-hydroxy-3-methyl-1-butoxy)-5-[4-(methylsulfonyl)phenyl]-3(2H)-pyridazinone (Example 39) for 2-(3,4-difluorophenyl)-4-(2-hydroxy-2-methyl-1-propoxy)-5-[4-(methylsulfonyl)phenyl]-3(2H)-pyridazinone.

### Example 43

### 2-(3-Chloro-4-fluorophenyl)-4-(4-hydroxy-3-methyl-1-butoxy)-5-[4-(aminosulfonyl)phenyl]-3(2H)-pyridazinone

The title compound may be prepared according to the method of Example 14, substituting 2-(3-chloro-4-fluorophenyl)-4-(4-hydroxy-3-methyl-1-butoxy)-5-[4-(methylsulfonyl)phenyl]-3(2H)-pyridazinone (Example 40) for 2-(3,4-difluorophenyl)-4-(2-hydroxy-2-methyl-1-propoxy)-5-[4-(methylsulfonyl)phenyl]-3(2H)-pyridazinone.

### Example 44

### 2-(3-Chlorophenyl)-4-(4-hydroxy-3-methyl-1-butoxy)-5-[4-(aminosulfonyl)phenyl]-3(2H)-pyridazinone

The title compound may be prepared according to the method of Example 14, substituting 2-(3-chlorophenyl)-4-(4-hydroxy-3-methyl-1-butoxy)-5-[4-(methylsulfonyl)phenyl]-3(2H)-pyridazinone (Example 41) for 2-(3,4-difluorophenyl)-4-(2-hydroxy-2-methyl-1-propoxy)-5-[4-(methylsulfonyl)phenyl]-3(2H)-pyridazinone.

### Example 45

### (S)-2-(4-Fluorophenyl)-4-(3-hydroxy-2-methyl-1-propoxy)-5-[4-(aminosulfonyl)phenyl]-3(2H)-pyridazinone

The title compound may be prepared according to the method of Example 17, substituting (S)-2-(4-fluorophenyl)-4-(3-t-butoxy-2-methyl-1-propoxy)-5-[4-(methylsulfonyl)phenyl]-3(2H)-pyridazinone for (S)-2-(3,4-difluorophenyl)-4-(3-t-butoxy-2-methyl-1-propoxy)-5-[4-(methylsulfonyl)phenyl]-3(2H)-pyridazinone.

### Example 46

### (R)-2-(4-Fluorophenyl)-4-(3-hydroxy-2-methyl-1-propoxy)-5-[4-(aminosulfonyl)phenyl]-3(2H)-pyridazinone

The title compound may be prepared according to the method of Example 18, substituting (R)-2-(4-fluorophenyl)-4-(3-t-butoxy-2-methyl-1-propoxy)-5-[4-(methylsulfonyl)phenyl]-3(2H)-pyridazinone for (R)-2-(3,4-difluorophenyl)-4-(3-t-butoxy-2-methyl-1-propoxy)-5-[4-(methylsulfonyl)phenyl]-3(2H)-pyridazinone.

### Example 47

### (S)-2-(3-Chloro-4-fluorophenyl)-4-(3-hydroxy-2-methyl-1-propoxy)-5-[4-(methylsulfonyl)phenyl]-3(2H)-pyridazinone

The title compound may be prepared according to the method of Example 15, substituting 2-(3-chloro-4-fluorophenyl)-4-hydroxy-5-[4-(methylsulfonyl)phenyl]-3(2H)-pyridazinone in place of 2-(3,4-difluorophenyl)-4-hydroxy-5-[4-(methylsulfonyl)phenyl-3(2H)-pyridazinone.

### Example 48

### (S)-2-(3-Chloro-4-fluorophenyl)-4-(3-hydroxy-2-methyl-1-propoxy)-5-[4-(aminosulfonyl)phenyl]-3(2H)-pyridazinone

The title compound can be prepared according to the method of Example 17, substituting (S)-2-(3-chloro-4-fluorophenyl)-4-(3-t-butoxy-2-methyl-1-propoxy)-5-[4-(methylsulfonyl)phenyl]-3(2H)-pyridazinone in place of (S)-2-(3,4-difluorophenyl)-4-(3-t-butoxy-2-methyl-1-propoxy)-5-[4-(methylsulfonyl)phenyl]-3(2H)-pyridazinone.

### Example 49

### (R)-2-(3-Chloro-4-fluorophenyl)-4-(3-hydroxy-2-methyl-1-propoxy)-5-[4-(methylsulfonyl)phenyl]-3(2H)-pyridazinone

The title compound may be prepared according to the method of Example 16, substituting 2-(3-chloro-4-fluorophenyl)-4-hydroxy-5-[4-(methylsulfonyl)phenyl]-3(2H)-pyridazinone in place of 2-(3,4-difluorophenyl)-4-hydroxy-5-[4-(methylsulfonyl)phenyl-3 (2H)-pyridazinone.

### Example 50

### (R)-2-(3-Chloro-4-fluorophenyl)-4-(3-hydroxy-2-methyl-1-propoxy)-5-[4-(aminosulfonyl)phenyl]-3(2H)-pyridazinone

The title compound may be prepared according to the method of Example 17, substituting (R)-2-(3-chloro-4-fluorophenyl)-4-(3-t-butoxy-2-methyl-1-propoxy)-5-[4-(methylsulfonyl)phenyl]-3(2H)-pyridazinone in place of (S)-2-(3,4-difluorophenyl)-4-(3-t-butoxy-2-methyl-1-propoxy)-5-[4-(methylsulfonyl)phenyl]-3(2H)-pyridazinone.

### Example 51

### (S)-2-(3-Chlorophenyl)-4-(3-hydroxy-2-methyl-1-propoxy)-5-[4-(methlsulfonyl)phenyl]-3(2H)-pyridazinone

The title compound may be prepared according to the method of Example 15, substituting 2-(3-chlorophenyl)-4-hydroxy-5-[4-(methylsulfonyl)phenyl]-3(2H)-pyridazinone in place of 2-(3,4-difluorophenyl)-4-hydroxy-5-[4-(methylsulfonyl)phenyl-3(2H)-pyridazinone.

### Example 52

### (S)-2-(3-Chlorophenyl)-4-(3-hydroxy-2-methyl-1-propoxy)-5-[4-(aminosulfonyl)phenyl]-3(2H)-pyridazinone

The title compound may be prepared according to the method of Example 17, substituting (S)-2-(3-chlorophenyl)-4-(3-t-butoxy-2-methyl-1-propoxy)-5-[4-(methylsulfonyl)phenyl]-3(2H)-pyridazinone in place of (S)-2-(3,4-difluorophenyl)-4-(3-t-butoxy-2-methyl-1-propoxy)-5-[4-(methylsulfonyl)phenyl]-3(2H)-pyridazinone.

### Example 53

### (R)-2-(3-Chlorophenyl)-4-(3-hydroxy-2-methyl-1-propoxy)-5-[4-(methylsulfonyl)phenyl]-3(2H)-pyridazinone

The title compound can be prepared according to the method of Example 16, substituting 2-(3-chlorophenyl)-4-hydroxy-5-[4-(methylsulfonyl)phenyl]-3(2H)-pyridazinone in place of 2-(3,4-difluorophenyl)-4-hydroxy-5-[4-(methylsulfonyl)phenyl]-3(2H)-pyridazinone.

### Example 54

### (R)-2-(3-Chlorophenyl)-4-(3-hydroxy-2-methyl-1-propoxy)-5-[4-(aminosulfonyl)phenyl]-3(2H)-pyridazinone

The title compound can be prepared according to the method of Example 17, substituting (R)-2-(3-chlorophenyl)-4-(3-t-butoxy-2-methyl-1-propoxy)-5-[4-(methylsulfonyl)phenyl]-3(2H)-pyridazinone in place of (S)-2-(3,4-difluorophenyl)-4-(3-t-butoxy-2-methyl-1-propoxy)-5-[4-(methylsulfonyl)phenyl]-3(2H)-pyridazinone.

### Example 55

### (S)-2-(2,2,2-Trifluoroethyl)-4-(3-hydroxy-2-methyl-1-propoxy)-5-[4-(methylsulfonyl)phenyl]-3(2H)-pyridazinone

The title compound may be prepared according to the method of Example 15, substituting 2-(2,2,2-trifluoroethyl)-4-hydroxy-5-[4-(methylsulfonyl)phenyl]-3(2H)-pyridazinone in place of 2-(3,4-difluorophenyl)-4-hydroxy-5-[4-(methylsulfonyl)phenyl-3(2H)-pyridazinone.

### Example 56

### (S)-2-(2,2,2-Trifluoroethyl)-4-(3-hydroxy-2-methyl-1-propoxy)-5-[4-(aminosulfonyl)phenyl]-3(2H)-pyridazinone

The title compound may be prepared according to the method of Example 35, substituting (R)-3-t-butoxy-2-methyl-1-propanol (Example 15A) in place of 2-methyl-1,2-propanediol. After Suzuki coupling with 4-[2-(tetrahydropyranyl)thio]phenylboronic acid, the resulting intermediate is treated with NCS and NH₄OH as in Example 35. This sulfonamide product is then treated with TFA (as in Example 15C) to cleave the t-butyl ether and provide the title compound.

### Example 57

### (R)-2-(2,2,2-Trifluoroethyl)-4-(3-hydroxy-2-methyl-1-propoxy)-5-[4-(methylsulfonyl)phenyl]-3(2H)-pyridazinone

The title compound may be prepared according to the method of Example 16, substituting 2-(2,2,2-trifluoroethyl)-4-hydroxy-5-[4-(methylsulfonyl)phenyl]-3(2H)-pyridazinone in place of 2-(3,4-difluorophenyl)-4-hydroxy-5-[4-(methylsulfonyl)phenyl-3(2H)-pyridazinone.

### Example 58

### (R)-2-(2,2,2-Trifluoroethyl)-4-(3-hydroxy-2-methyl-1-propoxy)-5-[4-(aminosulfonyl)phenyl]-3(2H)-pyridazinone

The title compound may be prepared according to the method of Example 56, substituting (S)-3-t-butoxy-2-methyl-1-propanol in place of (R)-3-t-butoxy-2-methyl-1-propanol.

### Example 59

### 2-(4-Fluorophenyl)-4-(3-hydroxy-2,2-dimethyl-1-propoxy)-5-[4-(methylsulfonyl)phenyl]-3(2H)-pyridazinone

The title compound may be prepared according to the sequence of reactions described in Example 22, substituting 4-fluorophenylhydrazine in place of 3,4-difluorophenylhydrazine and substituting neopentyl glycol in place of 3-methyl-1,3-butanediol.

### Example 60

### 2-(4-Fluorophenyl)-4-(3-hydroxy-2,2-dimethyl-1-propoxy)-5-[4-(aminosulfonyl)phenyl]-3(2H)-pyridazinone

The title compound may be prepared according to the method of Example 14, substituting 2-(4-fluorophenyl)-4-(3-hydroxy-2,2-dimethyl-1-propoxy)-5-[4-(methylsulfonyl)phenyl]-3(2H)-pyridazinone for 2-(3,4-difluorophenyl)-4-(2-hydroxy-2-methyl-1-propoxy)-5-[4-(methylsulfonyl)phenyl]-3(2H)-pyridazinone.

### Example 61

### 2-(3,4-Difluorophenyl)-4-(3-hydroxy-2,2-dimethyl-1-propoxy)-5-[4-(methylsulfonyl)phenyl]-3(2H)-pyridazinone

The title compound was prepared according to the sequence of reactions described in Example 22, substituting neopentyl glycol in place of 3-methyl-1,3-butanediol (yield: 300 mg, 71%). mp 161-162°C; ¹H NMR (300 MHz, DMSO-d₆) δ 0.72 (s, 6H), 3.05 (d, J = 6 Hz, 2H), 3.30 (s, 3H), 4.19 (s, 2H), 4.54 (t, J = 6 Hz, 1H), 7.52 (m, 1H), 7.62 (dd, J = 9 Hz, J = 9 Hz, 1H), 7.82 (ddd, J = 9 Hz, J = 9 Hz, J = 3 Hz, 1H), 7.92 (d, J = 9 Hz, 1H), 8.08 (d, J = 9 Hz, 2H), 8.21 (s, 1H); MS (DCI/NH₃) m/z 465 (M+H)⁺; Anal. calcd. for C₂₂H₂₂F₂N₂O₅S: C, 56.88; H, 4.77; N, 6.03. Found: C, 56.84; H, 4.83; N, 5.99.

### Example 62

### 2-(3,4-Difluorophenyl)-4-(3-hydroxy-2,2-dimethyl-1-propoxy)-5-[4-(aminosulfonyl)-phenyl]-3(2H)-pyridazinone

The title compound may be prepared according to the method of Example 14, substituting 2-(3,4-difluorophenyl)-4-(3-hydroxy-2,2-dimethyl-1-propoxy)-5-[4-(methylsulfonyl)phenyl]-3(2H)-pyridazinone for 2-(3,4-difluorophenyl)-4-(2-hydroxy-2-methyl-1-propoxy)-5-[4-(methylsulfonyl)phenyl]-3(2H)-pyridazinone.

### Example 63

### 2-(3-Chloro-4-fluorophenyl)-4-(3-hydroxy-2,2-dimethyl-1-propoxy)-5-[4-(methylsulfonyl)phenyl]-3-(2H)-pyridazinone

The title compound may be prepared according to the sequence of reactions described in Example 22, substituting 3-chloro-4-fluorophenylhydrazine in place of 3,4-difluorophenylhydrazine and substituting neopentyl glycol in place of 3-methyl-1,3-butanediol.

### Example 64

### 2-(3-Chloro-4-fluorophenyl)-4-(3-hydroxy-2,2-dimethyl-1-propoxy)-5-[4-(aminosulfonyl)phenyl]-3(2H)-pyridazinone

The title compound may be prepared according to the method of Example 14, substituting 2-(3-chloro-4-fluorophenyl)-4-(3-hydroxy-2,2-dimethyl-1-propoxy)-5-[4-(methylsulfonyl)phenyl]-3(2H)-pyridazinone for 2-(3,4-difluorophenyl)-4-(2-hydroxy-2-methyl-1-propoxy)-5-[4-(methylsulfonyl)phenyl]-3(2H)-pyridazinone.

### Example 65

### 2-(3-Chlorophenyl)-4-(3-hydroxy-2,2-dimethyl-1-propoxy)-5-[4-(methylsulfonyl)phenyl]-3(2H)-pyridazinone

The title compound may be prepared according to the sequence of reactions described in Example 22, substituting 3-chlorophenylhydrazine in place of 3,4-difluorophenylhydrazine and substituting neopentyl glycol in place of 3-methyl-1,3-butanediol.

### Example 66

### 2-(3-Chlorophenyl)-4-(3-hydroxy-2,2-dimethyl-1-propoxy)-5-[4-(aminosulfonyl)phenyl]-3(2H)-pyridazinone

The title compound may be prepared according to the method of Example 14, substituting 2-(3-chlorophenyl)-4-(3-hydroxy-2,2-dimethyl-1-propoxy)-5-[4-(methylsulfonyl)phenyl]-3(2H)-pyridazinone for 2-(3,4-difluorophenyl)-4-(2-hydroxy-2-methyl-1-propoxy)-5-[4-(methylsulfonyl)phenyl]-3(2H)-pyridazinone.

### Example 67

### N-[[4-[2-(4-Fluorophenyl)-4-(3-hydroxy-3-methyl-1-butoxy)-2H-pyridazin-3-on-5-yl]phenyl]sulfonyl]acetamide

A mixture of 2-(4-fluorophenyl)-4-(3-hydroxy-3-methyl-1-butoxy)-5-[4-(aminosulfonyl)phenyl]-3(2H)-pyridazinone (Example 21, 1 equivalent), acetic anhydride (3 equivalents), 4-(dimethylamino)pyridine (0.3 equivalents), and triethylamine (1.2 equivalents) is stirred at room temperature for 16 hours. The reaction mixture is partitioned between ethyl acetate and water. The organic layer is washed with brine then dried over MgSO₄ and filtered. The filtrate is concentrated in vacuo to give the title compound.

### Example 68

### N-[[4-[2-(4-Fluorophenyl)-4-(3-hydroxy-3-methyl-1-butoxy)-2H-pyridazin-3-on-5-yl]phenyl]sulfonyl]acetamide, sodium salt

To a suspension of N-[[4-[2-(4-Fluorophenyl)-4-(3-hydroxy-3-methyl-1-butoxy)-2H-pyridazin-3-on-5-yl]phenyl]sulfonyl]acetamide (Example 67, 1 equivalent) in absolute ethanol is added a solution of sodium hydroxide (1 equivalent) in absolute ethanol. The mixture is stirred at room temperature for 10 minutes and concentrated in vacuo. The residue is dried at high vacuum to provide the title compound.

### Example 69

### N-[[4-[2-(3,4-Difluorophenyl)-4-(2-hydroxy-2-methyl-1-propoxy)-2H-pyridazin-3-on-5-yl]phenyl]sulfonyl]acetamide

A mixture of 2-(3,4-difluorophenyl)-4-(2-hydroxy-2-methyl-1-propoxy)-5-[4-(aminosulfonyl)phenyl]-3(2H)-pyridazinone (Example 14, 1 equivalent), acetic anhydride (3 equivalents), 4-(dimethylamino)pyridine (0.3 equivalents), and triethylamine (1.2 equivalents) is stirred at room temperature for 16 hours. The reaction mixture is partitioned between ethyl acetate and water. The organic layer is washed with brine then dried over MgSO₄ and filtered. The filtrate is concentrated in vacuo to give the title compound.

### Example 70

### N-[[4-[2-(3,4-Difluorophenyl)-4-(2-hydroxy-2-methyl-1-propoxy)-2H-pyridazin-3-on-5-yl]phenyl]sulfonyl]acetamide, sodium salt

To a suspension of N-[[4-[2-(3,4-difluorophenyl)-4-(2-hydroxy-2-methyl-1-propoxy)-2H-pyridazin-3-on-5-yl]phenyl]sulfonyl]acetamide (Example 69, 1 equivalent) in absolute ethanol is added a solution of sodium hydroxide (1 equivalent) in absolute ethanol. The mixture is stirred at room temperature for 10 minutes and concentrated in vacuo. The residue is dried at high vacuum to provide the title compound.

### Example 71

### N-[[4-[2-(3-Chloro-4-fluorophenyl)-4-(2-hydroxy-2-methyl-1-propoxy)-2H-pyridazin-3-on-5-yl]phenyl]sulfonyl]acetamide

A mixture of 2-(3-chloro-4-fluorophenyl)-4-(2-hydroxy-2-methyl-1-propoxy)-5-[4-(aminosulfonyl)phenyl]-3(2H)-pyridazinone (Example 31, 1 equivalent), acetic anhydride (3 equivalents), 4-(dimethylamino)pyridine (0.3 equivalents), and triethylamine (1.2 equivalents) is stirred at room temperature for 16 hours. The reaction mixture is partitioned between ethyl acetate and water. The organic layer is washed with brine then dried over MgSO₄ and filtered. The filtrate is concentrated in vacuo to give the title compound.

### Example 72

### N-[[4-[2-(3-Chloro-4-fluorophenyl)-4-(2-hydroxy-2-methyl-1-propoxy)-2H-pyridazin-3-on-5-yl]phenyl]sulfonyl]acetamide, sodium salt

To a suspension of N-[[4-[2-(3-chloro-4-fluorophenyl)-4-(2-hydroxy-2-methyl-1-propoxy)-2H-pyridazin-3-on-5-yl]phenyl]sulfonyl]acetamide (Example 71, 1 equivalent) in absolute ethanol is added a solution of sodium hydroxide (1 equivalent) in absolute ethanol. The mixture is stirred at room temperature for 10 minutes and concentrated in vacuo. The residue is dried at high vacuum to provide the title compound.

### Example 73

### N-[[4-[2-(3-Chlorophenyl)-4-(2-hydroxy-2-methyl-1-propoxy)-2H-pyridazin-3-on-5-yl]phenyl]sulfonyl]acetamide

A mixture of 2-(3-chlorophenyl)-4-(2-hydroxy-2-methyl-1-propoxy)-5-[4-(aminosulfonyl)phenyl]-3(2H)-pyridazinone (Example 33, 1 equivalent), acetic anhydride (3 equivalents), 4-(dimethylamino)pyridine (0.3 equivalents), and triethylamine (1.2 equivalents) is stirred at room temperature for 16 hours. The reaction mixture is partitioned between ethyl acetate and water. The organic layer is washed with brine then dried over MgSO₄ and filtered. The filtrate is concentrated in vacuo to give the title compound.

### Example 74

### N-[[4-[2-(3-Chlorophenyl)-4-(2-hydroxy-2-methyl-1-propoxy)-2H-pyridazin-3-on-5-yl]phenyl]sulfonyl]acetamide, sodium salt

To a suspension of N-[[4-[2-(3-chlorophenyl)-4-(2-hydroxy-2-methyl-1-propoxy)-2H-pyridazin-3-on-5-yl]phenyl]sulfonyl]acetamide (Example 524, 1 equivalent) in absolute ethanol is added a solution of sodium hydroxide (1 equivalent) in absolute ethanol. The mixture is stirred at room temperature for 10 minutes and concentrated in vacuo. The residue is dried at high vacuum to provide the title compound.

### Example 75

### N-[[4-[2-(2,2,2-Trifluoroethyl)-4-(2-hydroxy-2-methyl-1-propoxy)-2H-pyridazin-3-on-5-yl]phenyl]sulfonyl]acetamide

A mixture of 2-(2,2,2-trifluoroethyl)-4-(2-hydroxy-2-methyl-1-propoxy)-5-[4-(aminosulfonyl)phenyl]-3(2H)-pyridazinone (Example 35, 1 equivalent), acetic anhydride (3 equivalents), 4-(dimethylamino)pyridine (0.3 equivalents), and triethylamine (1.2 equivalents) is stirred at room temperature for 16 hours. The reaction mixture is partitioned between ethyl acetate and water. The organic layer is washed with brine then dried over MgSO₄ and filtered. The filtrate is concentrated in vacuo to give the title compound.

### Example 76

### N-[[4-[2-(2,2,2-Trifluoroethyl)-4-(2-hydroxy-2-methyl-1-propoxy)-2H-pyridazin-3-on-5-yl]phenyl]sulfonyl]acetamide, sodium salt

To a suspension of N-[[4-[2-(2,2,2-trifluoroethyl)-4-(2-hydroxy-2-methyl-1-propoxy)-2H-pyridazin-3-on-5-yl]phenyl]sulfonyl]acetamide (Example 75, 1 equivalent) in absolute ethanol is added a solution of sodium hydroxide (1 equivalent) in absolute ethanol. The mixture is stirred at room temperature for 10 minutes and concentrated in vacuo. The residue is dried at high vacuum to provide the title compound.

### Example 77

### N-[[4-[2-(2,2,2-Trifluoroethyl)-4-(3-hydroxy-2,2-dimethyl-1-propoxy)-2H-pyridazin-3-on-5-yl]phenyl]sulfonyl]acetamide

A mixture of 2-(2,2,2-trifluoroethyl)-4-(3-hydroxy-2,2-dimethyl-1-propoxy)-5-[4-(aminosulfonyl)phenyl]-3(2H)-pyridazinone (Example 37, 1 equivalent), acetic anhydride (3 equivalents), 4-(dimethylamino)pyridine (0.3 equivalents), and triethylamine (1.2 equivalents) is stirred at room temperature for 16 hours. The reaction mixture is partitioned between ethyl acetate and water. The organic layer is washed with brine then dried over MgSO₄ and filtered. The filtrate is concentrated in vacuo to give the title compound.

### Example 78

### N-[[4-[2-(2,2,2-Trifluoroethyl)-4-(3-hydroxy-2,2-dimethyl-1-propoxy)-2H-pyridazin-3-on-5-yl]phenyl]sulfonyl]acetamide, sodium salt

To a suspension of N-[[4-[2-(2,2,2-trifluoroethyl)-4-(3-hydroxy-2,2-dimethyl-1-propoxy)-2H-pyridazin-3-on-5-yl]phenyl]sulfonyl]acetamide (Example 77, 1 equivalent) in absolute ethanol is added a solution of sodium hydroxide (1 equivalent) in absolute ethanol. The mixture is stirred at room temperature for 10 minutes and concentrated in vacuo. The residue is dried at high vacuum to provide the title compound.

### Example 79

### 2-(2,2,2-Trifluoroethyl)-4-(3-hydroxy-3-methyl-1-butoxy)-5-[4-(methylsulfonyl)phenyl]-3(2H)-pyridazinone

The title compound was prepared according to the sequence of reactions described in Example 22, substituting 2,2,2-trifluoroethylhydrazine in place of 3,4-difluorophenylhydrazine. ¹H NMR (300 MHz, CDCl₃) δ 1.25 (s, 6H), 1.88 (t, 2H, J = 9 Hz), 2.35 (br s, 1H), 3.15 (s, 3H), 4.55 (t, 2H, J = 7.5 Hz), 4.85 (q, 2H, J = 9 Hz), 7.75(d, 2H J = 9 Hz), 7.65 (s, 1H), 8.05 (d, 2H J = 9 Hz); MS (DCI/NH₃) m/z 435 (M+H)⁺; Analysis calculated for C₁₈H₂₁F₃N₂O₅S: C, 49.77; H, 4.87; N, 6.45. Found: C, 49.71; H, 4.90; N, 6.45.

### Example 80

### 2-(2,2,2-Trifluoroethyl)-4-(3-hydroxy-3-methyl-1-butoxy)-5-[4-(aminosulfonyl)phenyl]-3(2H)-pyridazinone

The title compound was prepared according to the method of Example 35, substituting 3-methyl-1,3-butanediol in place of 2-methyl-1,2-propanediol. ¹H NMR (300 MHz, CDCl₃) δ 1.85 (t, 2H, J = 6 Hz), 2.78 (s, 6H), 4.55 (t, 2H, J = 6 Hz), 4.85 (q, 2H, J = 9 Hz), 5.3 (s, 2H), 7.68 (d, 2H J = 9 Hz), 7.85 (s, 1H), 8.05 (d, 2H J = 9Hz), 8.45 (br s, 1H); MS (DCI/NH₃) m/z 436 (M+H)⁺; Analysis calculated for C₁₇H₂₀F₃N₃O₅S: C, 46.89; H, 4.62; N, 9.65. Found: C, 47.18; H, 4.93; N, 9.86.

### Example 81

### 2-(3,4-Dichlorophenyl)-4-(3-hydroxy-3-methyl-1-butoxy)-5-[4-(methylsulfonyl)phenyl]-3(2H)-pyridazinone

The title compound was prepared according to the sequence of reactions described in Example 22, substituting 3,4-dichlorophenylhydrazine in place of 3,4-difluorophenylhydrazine. mp 118-120°C; ¹H NMR (300 MHz, CDCl₃) δ 1.25 (s, 6H), 1.92 (t, J = 6 Hz, 2H), 3.13 (s, 3H), 4.07 (t, J = 6 Hz, 2H), 7.58 (d, J = 9 Hz, 1H), 7.59 (dd, J = 9 Hz, J = 2 Hz, 1H), 7.80 (d, J = 9 Hz, 2H), 7.87 (d, J = 2 Hz, 1H), 7.84 (s, 1H), 8.19 (d, J = 9 Hz, 2H); MS (DCI/NH₃) m/z 497 (M+H)⁺. Anal. calcd. for C₂₂H₂₂Cl₂N₂O₅S: C, 53.12; H, 4.45; N, 5.63. Found: C, 52.80; H, 4.53; N, 5.35.

### Example 82

### 2-[(3-Trifluoromethyl)phenyl]-4-(2-hydroxy-2-methyl-1-propoxy)-5-[4-(methylsulfonyl)phenyl]-3(2H)-pyridazinone

The title compound was prepared according to the sequence of reactions described in Example 22, substituting 3-(trifluoromethyl)phenylhydrazine in place of 3,4-difluorophenylhydrazine and substituting 2-methyl-1,2-propanediol (prepared by the LAH reduction of methyl 2-hydroxyisobutyrate) in place of 3-methyl-1,3-butanediol (yield: 200 mg, 75%). mp 143-144°C; ¹H NMR (300 MHz, CDCl₃) δ 1.20 (s, 6H), 3.13 (s, 3H), 4.11 (s, 2H), 7.64 (m, 2H), 7.84 (d, J = 9 Hz, 2H), 7.90 (d, J = 9 Hz, 1H), 7.97 (d, J = 9 Hz, 1H), 7.98 (s, 1H), 8.13 (d, J = 9 Hz, 2H); MS (DCI/NH₃) m/z 483 (M+H)⁺; Anal. calcd. for C₂₂H₂₁F₃N₂O₅S0.5C₄H₁₀O₂: C, 54.75; H, 4.79; N, 5.32. found: C, 55.15; H, 4.77; N, 5.09.

### Example 83

### 2-(3,4-Dichlorophenyl)-4-(2-hydroxy-2-methyl-1-propoxy)-5-[4-(methylsulfonyl)phenyl]-3(2H)-pyridazinone

The title compound was prepared according to the sequence of reactions described in Example 22, substituting 3,4-dichlorophenylhydrazine in place of 3,4-difluorophenylhydrazine and substituting 2-methyl-1,2-propanediol (prepared by the LAH reduction of methyl 2-hydroxyisobutyrate) in place of 3-methyl-1,3-butanediol (yield: 1.7 g, 75%). mp 108-110°C; ¹H NMR (300 MHz, DMSO-d₆) δ 0.96 (s, 6H), 3.38 (s, 3H), 4.20 (s, 2H), 4.52 (s, 1H), 7.68 (dd, J = 9 Hz, J = 3 Hz, 1H), 7.83 (d, J = 9 Hz, 1H), 7.78 (d, J = 9 Hz, 2H), 7.79 (d, J = 3 Hz, 1H), 8.04 (d, J = 9 Hz, 2H), 8.22 (s, 1H); MS (DCI/NH₃) m/z 483 (M+H)⁺; Anal. calcd. for C₂₁H₂₀Cl₂N₂O₅S: C, 52.18; H, 4.17; N, 5.79. Found: C, 52.41; H, 4.22; N, 5.53.

### Example 84 (reference example)

### 2-(3,4-Difluorophenyl)-4-(1-butoxy)-5-[4-(methylsulfonyl)phenyl]-3(2H)-pyridazinone

### Example 84A

### 2-(3,4-Difluorophenyl)-4-(1-butoxy)-5-chloro-3(2H)-pyridazinone

To a stirred, room temperature solution of n-butanol (0.81 g, 10.93 mmol, 1.1 equiv.) in THF (20 mL) was slowly added 1.0 M sodium bis(trimethylsilyl)amide in THF (12 mL, 12 mmol, 1.2 equiv.). The reaction mixture was stirred for 0.5 hours, then it was transferred to a solution of 2-(3,4-difluorophenyl)-4,5-dichloro-3(2H)-pyridazinone (2.88 g, 10.4 mmol, 1.0 equiv.) in THF (80 mL). The resulting reaction mixture was stirred for 0.5 hours at room temperature to provide the title compound (2.5 g, 79.4%).

### Example 84B

### 2-(3,4-Difluorophenyl)-4-(1-butoxy)-5-[4-(methylthio)phenyl]-3(2H)-pyridazinone

A slurry of palladium(II) acetate (9.0 mg, 0.04 mmol), triphenylphosphine (21.0 mg, 0.08 mmol) and isopropanol (1 mL) was stirred at room temperature for 10 minutes. To this mixture was added 2-(3,4-Difluorophenyl)-4-(1-butoxy)-5-chloro-3(2H)-pyridazinone (Example 84A, 0.63 g, 2 mmol), 4-(methylthio)benzeneboronic acid (0.403 mg, 2.4 mmol) and isopropanol (4 mL). A solution of K₃PO₄ (0.66 g, 3 mmol) in water (1 mL) was also added and the resulting reaction mixture was deoxygenated by bubbling nitrogen through it for 2 minutes. The reaction mixture was then stirred under a nitrogen atmosphere for 15 hours at 70 °C. The reaction mixture was then cooled to room temperature and water (15 mL) was added to provide a precipitate. The precipitate was collected by filtration and rinsed with water then hexane to give after drying the title compound (0.77 g, 95%).

### Example 84C

### 2-(3,4-Difluorophenyl)-4-(1-butoxy)-5-[4-(methylsulfonyl)phenyl]-3(2H)-pyridazinone

To a solution of 2-(3,4-difluorophenyl)-4-(1-butoxy)-5-[4-(methylthio)phenyl]-3(2H)-pyridazinone (Example 84B, 0.6 g, 1.5 mmol) in acetone (10 mL) at -20 °C was slowly added over 5 minutes a 32% solution of peracetic acid in acetic acid (3.75 mmol). The reaction mixture was allowed to warm to room temperature at which point water (30 mL) was added. Stirring was continued for 0.5 hours, then the precipitate was collected by filtration and washed with water to provide the title compound (0.61 g, 94%). mp 129-132°C; ¹H NMR (300 MHz, CDCl₃) δ 0.88 (t, J = 6 Hz, 3H), 1.20-1.36 (m, 2H), 1.54-1.68 (m, 2H), 3.14 (s, 3H), 4.52 (t, J = 6 Hz, 2H), 7.25-7.34 (m, 1H), 7.44-7.50 (m, 1H), 7.55-7.62 (m, 2H), 7.77-7.82 (m, 2H), 7.92 (s, 1H), 8.05-8.10 (m, 2H); MS (DCI/NH₃) m/z 435 (M+H)⁺; Anal. calcd. for C₂₁H₂₀F₂N₂O₄S: C, 58.06; H, 4.64; N, 6.44. Found: C, 57.82; H, 4.53; N, 6.31.

### Example 85

### 2-[3-(Trifluoromethyl)phenyl]-4-(3-hydroxy-3-methyl-1-butoxy)-5-[4-(methylsulfonyl)phenyl]-3(2H)-pyridazinone

The title compound was prepared according to the method of Example 84 substituting 3-methyl-1,3-butanediol in place of n-butanol and substituting 2-[3-(trifluoromethyl)phenyl]-4,5-dibromo-3(2H)-pyridazinone in place of 2-(3,4-difluorophenyl)-4,5-dichloro-3(2H)-pyridazinone to provide the title compound (1.2 g, 75%). mp 90-93 °C; ¹H NMR (300 MHz, CDCl₃) δ 1.14 (s, 6H), 1.90 (t, J = 6 Hz, 2H), 3.14 (s, 3H), 4.58 (t, J = 6 Hz, 2H), 7.60-7.70 (m, 2H), 7.79-7.84 (m, 2H), 7.94 (s, 1H), 7.88-7.98 (m, 2H), 8.06-8.12 (m, 2H); MS (DCI/NH₃) m/z 514 (M+H)⁺; Anal. calcd. for C₂₃H₂₃F₃N₂O₅S: C, 55.64; H, 4.67; N, 5.64. Found: C, 56.01; H, 4.83; N, 5.06.

### Prostaglandin Inhibition Determination

### Compound Preparation and Administration

For oral administration, test compounds were suspended on the day of use in 100% polyethyleneglycol (PEG 400) with a motorized homogenizer equipped with a Teflon-coated pestle (TRI-R Instrument, Jamaica, NY).

To compare the mean responses of the treatment groups, analysis of variance was applied. Percent inhibition values were determined by comparing the individual treatment mean values to the mean of the control group. Linear regression was used to estimate IC₅₀'s/ED₅₀'s in appropriate assays.

### EIA Determination of Prostaglandins

EIA reagents for prostaglandin determination were purchased from Perseptive Diagnostics, (Cambridge, MA). Prostaglandin E₂ (PGE₂) levels in lavage fluids were determined after the samples were dried under nitrogen and reconstituted with assay buffer. PGE₂ levels in enzyme assays or cell culture media were measured against standards prepared in the same milieu. The immunoassays were conducted as recommended by the manufacturer. The EIA was conducted in 96 well microtiter plates (Nunc Roskilde, Denmark) and optical density was measured using a microplate reader (Vmax, Molecular Devices Corp., Menlo Park, CA).

### Recombinant Human PGHS-1 and PGHS-2 Enzyme Assays

Inhibition of prostaglandin biosynthesis in vitro was evaluated using recombinant human Cox-1 (r-hu Cox1) and Cox-2 (r-hu Cox-2) enzyme assays. Representative compounds dissolved in DMSO (3.3% v/v) were preincubated with microsomes from recombinant human PGHS-1 or PGHS-2 expressed in the baculovirus/Sf9 cell system (Gierse, J. K., Hauser,S. D., Creely, D. P., Koboldt, C., Rangwala, S., H., Isakson, P. C., and Seibert, K. Expression and selective inhibition of the constituitive and inducible forms of cyclooxygenase, Biochem J. 1995, 305: 479.), together with the cofactors phenol (2 mM) and hematin (1 µM) for 60 minutes prior to the addition of 10 µM arachidonic acid. The reaction was allowed to run for 2.5 minutes at room temperature prior to quenching with HCl and neutralization with NaOH. PGE₂ production in the presence and absence of the drug was determined by EIA analysis. The EIA was conducted in 96 well microtiter plates (Nunc Roskilde, Denmark) and optical density was measured using a microplate reader (Vmax, Molecular Devices Corp., Menlo Park, CA). EIA reagents for prostaglandin determination were purchased from Perseptive Diagnostics (Cambridge, MA). PGE₂ levels were measured against standards prepared in the same milieu. The immunoassays were conducted as recommended by the manufacturer.

The data illustrating the inhibition of prostaglandin biosynthesis in vitro by compounds of this invention is shown in Table 1. The compounds are designated by the Example Number. Column 2 shows Cox-1 percent inhibition at the particular micromolar dose level and Column 3 shows Cox-2 percent inhibition at the particular nanomolar dose level. Values for Cox-1 and Cox-2 inhibition that are parenthetical indicate IC₅₀ values.

**Table 1**

| Example Number | RHUCX1 % Inh. at Dose (µM) | RHUCX2 % Inh. at Dose (µM) |
|---|---|---|
| 11 | 46 @ 100 31 @ 10 | (0.18) |
| 12 | 10 @ 100 | (0.470) |
| 26 | 0 @ 100 | (0.71) |
| 22 | (3.68) | (0.49) |
| 29 | 33 @ 100 | (0.81) |
| 77 | (3.4) | (0.72) |

### IL-1β Induced PGE₂ Production in WISH Cells

Human amnionic WISH cells were grown to 80% confluence in 48 well plates. Following removal of the growth medium and two washings with Gey's Balanced Salt Solutn, 5 ng IL-1β/ml (UBI, Lake Placid, NY) was added to the cells with or without test compound in DMSO (0.01% v/v) in Neuman-Tytell Serumless Medium (GIBCO, Grand Island, NY). Following an 18 hour incubation to allow for the maximal induction of PGHS-2, the conditioned medium was removed and assayed for PGE₂ content by EIA analysis as described above.

The data illustrating the inhibition of prostaglandin biosynthesis in vitro by compounds of this invention is shown in Table 2. WISH cell values indicate percent inhibition at the particular micromolar dose level while parenthetical values indicate IC₅₀ values.

### Human Whole Platelet Cyclooxygenase-1 Assay (HWCX)

Blood from normal healthy volunteers is collected into tubes containing ACD (acid citrate dextrose) as the anticoagulant. This blood is centrifuged at 175 x g to prepare platelet rich plasma. The platelet rich plasma is then centrifuged at 100 x g to pellet the white blood cells, leaving the platelets in the supernatant. The supernatant is layered on a cushion of 0.7 mL of 10% bovine serum albumin in Tyrodes solution (Gibco; Grand Island, NY) and then centrifuged at 1000 x g. The resulting supernatant from this centrifugation is then removed and 11 mL of Tyrodes solution is added to the remaining pellet of platelets. The platelets are then aliquoted at 120 µl into a 96 well plate. Experimental compounds are added and allowed to pre-incubate for 10 minutes. At the end of this pre-incubation period, the calcium ionophore A23187 is added to a final concentration of 8.8 µM and the incubation is continued for ten minutes. The reaction is stopped by adding cold 6 mM EDTA, the incubation mixture is centrifuged at 220 x g, and the supernatants are then analyzed for thromboxane using a commercial kit from Cayman Chemical (Ann Arbor, MI).

**Table 2**

| Example Numbers | HWPX % Inhib. at Dose (µM) | Wish % Inhib. at Dose (µM) |
|---|---|---|
| 11 | (2.04) | (0.089) |
| 12 | (22.4) | (0.15) |
| 26 | 50 @ 100 | (0.44) |
| 22 | (0.32) | (0.04) |
| 29 | (0.5) | (0.108) |
| 77 | (3.5) | (0.054) |

### Carrageenan Induced Paw Edema (CPE) in Rats

Hindpaw edema was induced in male rats as described by Winter et al., Proc. Soc. Exp. Biol. Med., 1962, 111, 544. Briefly, male Sprague-Dawley rats weighing between 170 and 190 g were administered test compounds orally 1 hour prior to the subplantar injection of 0.1 ml of 1% sodium carrageenan (lambda carrageenan, Sigma Chemical Co., St Louis, MO) into the right hindpaw. Right paw volumes (ml) were measured immediately following injection of carrageenan for baseline volume measurements using a Buxco plethysmograph (Buxco Electronics, Inc., Troy, NY). Three hours after the injection of carrageenan, right paws were remeasured and paw edema calculated for each rat by subtracting the zero time reading from the 3 hour reading. Data are reported as mean percent inhibition +/- SEM. Statistical significance of results was analyzed by Dunnetts multiple comparison test where p< 0.05 was considered statistically significant.

The data illustrating the inhibition of prostaglandin biosynthesis in vivo by the compounds of this invention is shown in Table 3. Values reported are percent inhibition at 10 milligrams per kilogram body weight.

Carrageenan induced air pouch prostaglandin biosynthesis model (CAP) Air pouches are formed in the backs of male Sprague Dawley rats by injecting 20 mL of sterile air on day 0. Three days later the pouch was reinflated with an additional 10 mL of sterile air. On day 7, 1 mL of saline containing 0.2 % lambda carrageenan (Sigma Chemical Co.) is injected into the pouch to induce the inflammatory reaction that is characterized by the release of prostaglandins. Test compounds are dosed at 0.1 to 10 mg/kg 30 minutes prior to carrageenan. Four hours after the carrageenan injection the pouch is lavaged and levels of prostaglandins are determined by enzyme immuno-assay using commercially available kits. Percent inhibitions are calculated by comparing the response in animals which have received vehicle to those which received compound. Values for Cox-2 inhibition that are parenthetical indicate ED₅₀ values.

The data illustrating the inhibition of prostaglandin biosynthesis in vivo by the compounds of this invention is shown in Table 3. Values reported are percent inhibition at 10 milligrams per kilogram body weight for CPE tests and at 3 milligrams per kilogram body weight for CAP testing.

**Table 3**

| Example Numbers | CPE % Inhib. @ 10 mpk | CAP % Inhib. @ 3 mpk |
|---|---|---|
| 11 | 47 | 77.5 ED₅₀ = 0.57 |
| 12 | ED₃₀=0.82 | 80.5 EDso = 0.7 |
| 26 | 33 | ED₅₀=1.4 |
| 22 | 41 | ED₅₀=0.9 |
| 29 | 26 | 75 |
| 77 | 40 | ED₅₀=1.4 |

### Human whole blood assays

### Cyclooxygenase-1

Heparin anticoagulatd blood was incubated with drugs dissolved in DMSO. The samples are incubated at 37 degrees Celsius for 4.5 hours after which calcium ionophore at a final concentration of 30 µM was added and the mixture allowed to incubate for 30 minutes. The reaction was stopped with the addition of EGTA and cold methanol (50% final concentration). After 18 hours at -70 degrees Celsius, the plates were centrifuged and supernatants analyzed for TXB₂.

### Cyclooxygenase-2

Heparin anticoagulated blood was incubated with drugs dissolved in DMSO. The samples are incubated at 37 degrees Celsius for 15 minutes. E. coli lipopolysaccaride (LPS) 5 µg/ml was then added and the samples incubated for 5 hours. The reaction was stopped with the addition of EGTA and cold methanol (50% final concentration). After 18 hours at -70 degrees Celsius, the plates were centrifuged and supernatants analyzed for TXB₂.
Example 12- Human whole blood Cox-1 IC₅₀ = 29.12 micromolar
Example 12-Human whole blood Cox-2 IC₅₀ = 0.47 micromolar
Example 12- Human whole blood Cox-1 = 55.5% at 30 micromolar
Example 12- Human whole blood Cox-2 = 85% at 0.03 micromolar

The 4-hydroxyalkoxy-3(2H)-pyridazinone Cox-2 inhibition and in vivo activity has been found to be suprisingly good. In addition, when compared to the 4-alkoxy-3(2H)-pyridazinones, the hydroxyalkoxy compounds typically possess a superior oral pharmacokinetic profile, such as better plasma half-life, plasma concentration maxima, and area under the curve. For example, Example 11 shows a 14 day plasma micromolar concentration (based on 10mg/kg dosage) of 0.0 while Example 12 shows a 3.43 micromolar level. In addition, the half life of Rat IV (3mg/kg) for Example 11 was 2.9 while Example 12 showed 5.2. The AUC value (micromolar/hour) for Example 11 was 1.8 while Example 12 was 69.

It is anticipated that the 4-hydroxyalkoxy compounds are preferred to parent alkoxy compounds with regard to once-a-day dosing to achieve predictable exposure levels across a wide range of doses while producing an antiinflammatory effect.

### Pharmaceutical Compositions

The present invention also provides pharmaceutical compositions which comprise compounds of the present invention formulated together with one or more non-toxic pharmaceutically acceptable carriers. The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the procedures and judgements well known to one skilled in the art. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

The compounds of the present invention may be potentially useful in the treatment of several illness or disease states such as inflammatory diseases, dysmennorhea, asthma, premature labor, adhesions and in particular pelvic adhesions, osteoporosis, and ankylosing spondolitis. Current Drugs Ltd, ID Patent Fast Alert, AG16, May 9, 1997.

The compounds of the present invention may also be potentially useful in the treatment of cancers, and in particular, colon cancer. Proc. Natl. Acad. Sci., 94, pp. 3336-3340, 1997.

The compounds of the present invention may be useful by providing a pharmaceutical composition for inhibiting prostaglandin biosynthesis comprising a therapeutically effective amount of a compound of formula I or a pharmaceutically acceptable salt, ester, or prodrug thereof, and a pharmaceutically acceptable carrier.

In addition, the compounds of the present invention may be useful by providing a method for inhibiting prostaglandin biosynthesis comprising administering to a mammal in need of such treatment a therapeutically effective amount of a compound of formula I or a pharmaceutically acceptable salt, ester, or prodrug thereof.

In addition, the compounds of the present invention may be useful by providing a method for treating pain, fever, inflamation, rheumatoid arthritis, osteoarthritis, adhesions, and cancer comprising administering to a mammal in need of such treatment a therapeutically effective amount of a compound of formula I.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (such as, for example, cottonseed, groundnut, corn, germ, olive, castor, sesame oils, and the like), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, such as, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, such as, for example, a solution in 1,3-butanediol. Among the acceptable vehicles and solvents that may be employed are water, Ringer's solution, isotonic sodium chloride solution, and the like. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectable preparations.

The injectable formulations can be sterilized by any method known in the art, such as, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsulated matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable nonirritating excipients or carriers such as cocoa butter, polyethylene glycol or a suppository wax which are solid at ambient temperature but liquid at body temperature and thus melt in the rectum or vaginal cavity and release the active compound.

Solid dosage forms for oral administration include capsules, tablets, pills, powders, and granules. In such solid dosage forms, the active compound is usually mixed with at least one inert, pharmaceutically acceptable excipient or carrier such as, for example, sodium citrate or dicalcium phosphate and/or a) fillers or extenders such as, for example, starches, lactose, sucrose, glucose, mannitol, and silicic acid, b) binders such as, for example, carboxymethylcellulose, alginates, gelatin, polyvinylpyrrolidinone, sucrose, and acacia, c) humectants such as, for example, glycerol, d) disintegrating agents such as, for example, agar-agar, calcium carbonate, potato or tapioca starch, alginic acid, certain silicates, and sodium carbonate, e) solution retarding agents such as, for example, paraffin, f) absorption accelerators such as, for example, quaternary ammonium compounds, g) wetting agents such as, for example, cetyl alcohol and glycerol monostearate, h) absorbents such as, for example, kaolin and bentonite clay, and) lubricants such as, for example, talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients such as, for example, lactose or milk sugar as well as high molecular weight polyethylene glycols and the like.

Solid compositions of a similar type may also be employed as fillers in soft and hard-filled gelatin capsules using excipients such as, for example, lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulation art. In such solid dosage forms the active compound may be admixed with at least one inert diluent such as, for example, sucrose, lactose or starch. Such dosage forms may also comprise, as is normal practice, additional substances other than inert diluents, e.g., tableting lubricants and other tableting aids such as, for example, magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as, for example, animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as, for example, lactose, talc, silicic acid, aluminum hydroxide, calcium silicates and polyamide powder, or mixtures of these substances. Sprays can additionally contain customary propellants such as chlbrofluorohydrocarbons.

Transdermal patches have the added advantage of providing controlled delivery of a compound to the body. Such dosage forms can be made by dissolving or dispensing the compound in a suitable medium. Absorption enhancers can also be used to increase the flux of the compound across the skin. The rate can be controlled by either providing a rate controlling membrane or by dispersing the compound in a polymer matrix or gel.

According to the methods of treatment using the compounds of the present invention, a patient, such as a human or mammal, is treated by administering to the patient a therapeutically effective amount of a compound of the invention, in such amounts and for such time as is necessary to achieve the desired result. By a "therapeutically effective amount" of a compound of the invention is meant a sufficient amount of the compound to provide the relief desired, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.001 to about 1000 mg/kg body weight daily or more preferably from about 0.1 to about 100 mg/kg body weight for oral administration or 0.01 to about 10 mg/kg for parenteral administration daily. Single dose compositions may contain such amounts or submultiples thereof to make up the daily dose.

The amount of active ingredient that may be combined with the carrier materials to produce a single dosage form will vary depending upon the host treated and the particular mode of administration.

The reagents required for the synthesis of the compounds of the invention are readily available from a number of commercial sources such as Aldrich Chemical Co. (Milwaukee, WI, USA); Sigma Chemical Co. (St. Louis, MO, USA); and Fluka Chemical Corp. (Ronkonkoma, NY, USA); Alfa Aesar (Ward Hill, MA 01835-9953); Eastman Chemical Company (Rochester, New York 14652-3512); Lancaster Synthesis Inc. (Windham, NH 03087-9977); Spectrum Chemical Manufacturing Corp. (Janssen Chemical) (New Brunswick, NJ 08901); Pfaltz and Bauer (Waterbury, CT. 06708). Compounds which are not commercially available can be prepared by employing known methods from the chemical literature.

## Claims

1. A compound selected from the group consisting of
2-(4-Fluorophenyl)-4-(4-hydroxy-2-methyl-1-butoxy)-5-[4-(methylsulfonyl)phenyl]-3(2H)-pyridazinone;
2-(4-Fluorophenyl)-4-(3-hydroxy-3-methyl-1-butoxy)-5-[4-(aminosulfonyl)phenyl]-3(2H)-pyridazinone;
2-(3,4-Difluorophenyl)-4-(3-hydroxy-3-methyl-1-butoxy)-5-[4-(methylsulfonyl)phenyl]-3(2H)-pyridazinone;
2-(3,4-Difluorophenyl)-4-(3-hydroxy-3-methyl-1-butoxy)-5-[4-(aminosulfonyl)phenyl]-3(2H)-pyridazinone;
2-(3-Chloro-4-fluorophenyl)-4-(3-hydroxy-3-methyl-1-butoxy)-5-[4-(methylsulfonyl)phenyl]-3(2H)-pyridazinone;
2-(3-Chloro-4-fluorophenyl)-4-(3-hydroxy-3-methyl-1-butoxy)-5-[4-(aminosulfonyl)phenyl]-3(2H)-pyridazinone;
2-(3-Chlorophenyl)-4-(3-hydroxy-3-methyl-1-butoxy)-5-[4-(methylsulfonyl)phenyl]-3(2H)-pyridazinone;
2-(3-Chlorophenyl)-4-(3-hydroxy-3-methyl-1-butoxy)-5-[4-(aminosulfonyl)phenyl]-3(2H)-pyridazinone;
2-(4-Fluorophenyl)-4-(2-hydroxy-2-methyl-1-propoxy)-5-[4-(methylsulfonyl)phenyl]-3(2H)-pyridazinone;
2-(4-Fluorophenyl)-4-(2-hydroxy-2-methyl-1-propoxy)-5-[4-(aminosulfonyl)phenyl]-3(2H)-pyridazinone;
2-(3-Chloro-4-fluorophenyl)-4-(2-hydroxy-2-methyl-1-propoxy)-5-[4-(methylsulfonyl)phenyl]-3(2H)-pyridazinone;
2-(3-Chloro-4-fluorophenyl)-4-(2-hydroxy-2-methyl-1-propoxy)-5-[4-(aminosulfonyl)phenyl]-3(2H)-pyridazinone;
2-(3-Chlorophenyl)-4-(2-hydroxy-2-methyl-1-propoxy)-5-[4-(methylsulfonyl)phenyl]-3(2H)-pyridazinone;
2-(3-Chlorophenyl)-4-(2-hydroxy-2-methyl-1-propoxy)-5-[4-(aminosulfonyl)phenyl]-3(2H)-pyridazinone;
2-(4-Fluorophenyl)-4-(4-hydroxy-3-methyl-1-butoxy)-5-[4-(aminosulfonyl)phenyl]-3(2H)-pyridazinone;
2-(3,4-Difluorophenyl)-4-(4-hydroxy-3-methyl-1-butoxy)-5-[4-(methylsulfonyl)phenyl]-3(2H)-pyridazinone;
2-(3-Chloro-4-fluorophenyl)-4-(4-hydroxy-3-methyl-1-butoxy)-5-[4-(methylsulfonyl)phenyl]-3(2H)-pyridazinone;
2-(3-Chlorophenyl)-4-(4-hydroxy-3-methyl-1-butoxy)-5-[4-(methylsulfonyl)phenyl]-3(2H)-pyridazinone;
2-(3,4-Difluorophenyl)-4-(4-hydroxy-3-methyl-1-butoxy)-5-[4-(aminosulfonyl)phenyl]-3(2H)-pyridazinone;
2-(3-Chloro-4-fluorophenyl)-4-(4-hydroxy-3-methyl-1-butoxy)-5-[4-(aminosulfonyl)phenyl]-3(2H)-pyridazinone;
2-(3-Chlorophenyl)-4-(4-hydroxy-3-methyl-1-butoxy)-5-[4-(aminosulfonyl)phenyl]-3(2H)-pyridazinone;
(S)-2-(4-Fluorophenyl)-4-(3-hydroxy-2-methyl-1-propoxy)-5-[4-(aminosulfonyl)phenyl]-3(2H)-pyridazinone;
(R)-2-(4-Fluorophenyl)-4-(3-hydroxy-2-methyl-1-propoxy)-5-[4-(aminosulfonyl)phenyl]-3(2H)-pyridazinone;
(S)-2-(3-Chloro-4-fluorophenyl)-4-(3-hydroxy-2-methyl-1-propoxy)-5-[4-(methylsulfonyl)phenyl]-3(2H)-pyridazinone;
(S)-2-(3-Chloro-4-fluorophenyl)-4-(3-hydroxy-2-methyl-1-propoxy)-5-[4-(aminosulfonyl)phenyl]-3(2H)-pyridazinone;
(R)-2-(3-Chloro-4-fluorophenyl)-4-(3-hydroxy-2-methyl-1-propoxy)-5-[4-(methylsulfonyl)phenyl]-3(2H)-pyridazinone;
(R)-2-(3-Chloro-4-fluorophenyl)-4-(3-hydroxy-2-methyl-1-propoxy)-5-[4-(aminosulfonyl)phenyl]-3(2H)-pyridazinone;
(S)-2-(3-Chlorophenyl)-4-(3-hydroxy-2-methyl-1-propoxy)-5-[4-(methylsulfonyl)phenyl]-3(2H)-pyridazinone;
(S)-2-(3-Chlorophenyl)-4-(3-hydroxy-2-methyl-1-propoxy)-5-[4-(aminosulfonyl)phenyl]-3(2H)-pyridazinone;
(R)-2-(3-Chlorophenyl)-4-(3-hydroxy-2-methyl-1-propoxy)-5-[4-(methylsulfonyl)phenyl]-3(2H)-pyridazinone;
(R)-2-(3-Chlorophenyl)-4-(3-hydroxy-2-methyl-1-propoxy)-5-[4-(aminosulfonyl)phenyl]-3(2H)-pyridazinone;
2-(4-Fluorophenyl)-4-(3-hydroxy-2,2-dimethyl-1-propoxy)-5-[4-(methylsulfonyl)phenyl]-3(2H)-pyridazinone;
2-(4-Fluorophenyl)-4-(3-hydroxy-2,2-dimethyl-1-propoxy)-5-[4-(aminosulfonyl)phenyl]-3(2H)-pyridazinone;
2-(3,4-Difluorophenyl)-4-(3-hydroxy-2,2-dimethyl-1-propoxy)-5-[4-(methylsulfonyl)phenyl]-3(2H)-pyridazinone;
2-(3,4-Difluorophenyl)-4-(3-hydroxy-2,2-dimethyl-1-propoxy)-5-[4-(aminosulfonyl)-phenyl]-3(2H)-pyridazinone;
2-(3-Chloro-4-fluorophenyl)-4-(3-hydroxy-2,2-dimethyl-1-propoxy)-5-[4-(methylsulfonyl)phenyl]-3(2H)-pyridazinone;
2-(3-Chloro-4-fluorophenyl)-4-(3-hydroxy-2,2-dimethyl-1-propoxy)-5-[4-(aminosulfonyl)phenyl]-3(2H)-pyridazinone;
2-(3-Chlorophenyl)-4-(3-hydroxy-2,2-dimethyl-1-propoxy)-5-[4-(methylsulfonyl)phenyl]-3(2H)-pyridazinone;
2-(3-Chlorophenyl)-4-(3-hydroxy-2,2-dimethyl-1-propoxy)-5-[4-(aminosulfonyl)phenyl]-3(2H)-pyridazinone;
2-(3,4-Dichlorophenyl)-4-(3-hydroxy-3-methyl-1-butoxy)-5-[4-(methylsulfonyl)phenyl]-3(2H)-pyridazinone;
2-[(3-Trifluoromethyl)phenyl]-4-(2-hydroxy-2-methyl-1-propoxy)-5-[4-(methylsulfonyl)phenyl]-3(2H)-pyridazinone;
2-(3,4-Dichlorophenyl)-4-(2-hydroxy-2-methyl-1-propoxy)-5-[4-(methylsulfonyl)phenyl]-3(2H)-pyridazinone; and
2-[3-(Trifluoromethyl)phenyl]-4-(3-hydroxy-3-methyl-1-butoxy)-5-[4-(methylsulfonyl)phenyl]-3(2H)-pyridazinone;
or a pharmaceutically acceptable salt or ester thereof.

2. A compound selected from the group consisting of
2-(2,2,2-Trifluoroethyl)-4-(2-hydroxy-2-methyl-1-propoxy)-5-[4-(methylsulfonyl)phenyl]-3(2H)-pyridazinone;
2-(2,2,2-Trifluoroethyl)-4-(2-hydroxy-2-methyl-1-propoxy)-5-[4-(aminosulfonyl)phenyl]-3(2H)-pyridazinone;
2-(2,2,2-Trifluoroethyl)-4-(3-hydroxy-2,2-dimethyl-1-propoxy)-5-[4-(methylsulfonyl)phenyl]-3(2H)-pyridazinone;
2-(2,2,2-Trifluoroethyl)-4-(3-hydroxy-2,2-dimethyl-1-propoxy)-5-[4-(aminosulfonyl)phenyl]-3(2H)-pyridazinone;
(S)-2-(2,2,2-Trifluoroethyl)-4-(3-hydroxy-2-methyl-1-propoxy)-5-[4-(methylsulfonyl)phenyl]-3(2H)-pyridazinone;
(S)-2-(2,2,2-Trifluoroethyl)-4-(3-hydroxy-2-methyl-1-propoxy)-5-[4-(aminosulfonyl)phenyl]-3(2H)-pyridazinone;
(R)-2-(2,2,2-Trifluoroethyl)-4-(3-hydroxy-2-methyl-1-propoxy)-5-[4-(methylsulfonyl)phenyl]-3(2H)-pyridazinone;
(R)-2-(2,2,2-Trifluoroethyl)-4-(3-hydroxy-2-methyl-1-propoxy)-5-[4-(aminosulfonyl)phenyl]-3(2H)-pyridazinone;
2-(2,2,2-Trifluoroethyl)-4-(3-hydroxy-3-methyl-1-butoxy)-5-[4-(methylsulfonyl) phenyl]-3(2H)-pyridazi none; and
2-(2,2,2-Trifluoroethyl)-4-(3-hydroxy-3-methyl-1-butoxy)-5-[4-(aminosulfonyl)phenyl]-3(2H)-pyridazinone;
or a pharmaceutically acceptable salt or ester thereof.

3. A compound selected from the group consisting of
N-[[4-[2-(4-Fluorophenyl)-4-(3-hydroxy-3-methyl-1-butoxy)-2H-pyridazin-3-on-5-yl]phenyl]sulfonyl]acetamide;
N-[[4-[2-(4-Fluorophenyl)-4-(3-hydroxy-3-methyl-1-butoxy)-2H-pyridazin-3-on-5-yl]phenyl]sulfonyl]acetamide, sodium salt;
N-[[4-[2-(3,4-Difluorophenyl)-4-(2-hydroxy-2-methyl-1-propoxy)-2H-pyridazin-3-on-5-yl]phenyl]sulfonyl]acetamide;
N-[[4-[2-(3,4-Difluorophenyl)-4-(2-hydroxy-2-methyl-1-propoxy)-2H-pyridazin-3-on-5-yl]phenyl]sulfonyl]acetamide, sodium salt;
N-[[4-[2-(3-Chloro-4-fluorophenyl)-4-(2-hydroxy-2-methyl-1-propoxy)-2H-pyridazin-3-on-5-yl]phenyl]sulfonyl]acetamide;
N-[[4-[2-(3-Chloro-4-fluorophenyl)-4-(2-hydroxy-2-methyl-1-propoxy)-2H-pyridazin-3-on-5-yl]phenyl]sulfonyl]acetamide, sodium salt;
N-[[4-[2-(3-Chlorophenyl)-4-(2-hydroxy-2-methyl-1-propoxy)-2H-pyridazin-3-on-5-yl]phenyl]sulfonyl]acetamide;
N-[[4-[2-(3-Chlorophenyl)-4-(2-hydroxy-2-methyl-1-propoxy)-2H-pyridazin-3-on-5-yl]phenyl]sulfonyl]acetamide, sodium salt;
N-[[4-[2-(2,2,2-Trifluoroethyl)-4-(2-hydroxy-2-methyl-1-propoxy)-2H-pyridazin-3-on-5-yl]phenyl]sutfonyl]acetamide;
N-[[4-[2-(2,2,2-Trifluoroethyl)-4-(2-hydroxy-2-methyl-1-propoxy)-2H-pyridazin-3-on-5-yl]phenyl]sulfonyl]acetamide, sodium salt;
N-[[4-[2-(2,2,2-Trifluoroethyl)-4-(3-hydroxy-2,2-dimethyl-1-propoxy)-2H-pyridazin-3-on-5-yl]phenyl]sulfonyl]acetamide; and
N-[[4-[2-(2,2,2-Trifluoroethyl)-4-(3-hydroxy-2,2-dimethyl-1-propoxy)-2H-pyridazin-3-on-5-yl]phenyl]sulfonyl]acetamide, sodium salt;
or a pharmaceutically acceptable salt or ester thereof

4. 2-(3,4-Difluorophenyl)-4-(3-hydroxy-3-methyl-1-butoxy)-5-[4-(methylsulfonyl)phenyl]-3(2H)-pyridazinone or a pharmaceutically acceptable salt or ester thereof.

5. A pharmaceutical composition comprising a therapeutically effective amount of a compound of any one of claims 1-4 and a pharmaceutically acceptable carrier.

6. A compound of any one of claims 1-4 for use as a therapeutic agent.

7. Use of a compound of any one of claims 1-4 for manufacturing a medicament for inhibiting prostaglandin biosynthesis in a mammal in need of such treatment.

8. Use of a compound of any one of claims 1-4 for manufacturing a medicament for treating pain, fever, inflammation, rheumatoid arthritis, osteoarthritis, adhesions, and cancer.

## Patentansprüche

1. Eine Verbindung gewählt aus der Gruppe bestehend aus
2-(4-Fluorphenyl)-4-(4-hydroxy-2-methyl-l-butoxy)-5-[4-(methylsulfonyl)phenyl]-3(2H)-pyridazinon;
2-(4-Fluorphenyl)-4-(3-hydroxy-3-methyl-1-butoxy)-5-[4-(aminosulfonyl)phenyl]-3(2H)-pyridazinon;
2-(3,4-Difluorphenyl)-4-(3-hydroxy-3-methyl-1-butoxy)-5-[4-(methylsulfonyl)phenyl]-3(2H)-pyridazinon;
2-(3,4-Difluorphenyl)-4-(3-hydroxy-3-methyl-1-butoxy)-5-[4-(aminosulfonyl)phenyl]-3(2H)-pyridazinon;
2-(3-Chlor-4-fluorphenyl)-4-(3-hydroxy-3-methyl-1-butoxy)-5-[4-(methylsulfonyl)phenyl]-3(2H)-pyridazinon;
2-(3-Chlor-4-fluorphenyl)-4-(3-hydroxy-3-methyl-1-butoxy)-5-[4-(aminosulfonyl)phenyl]-3(2H)-pyridazinon;
2-(3-Chlorphenyl)-4-(3-hydroxy-3-methyl-1-butoxy)-5-[4-(methylsulfonyl)phenyl]-3(2H)-pyridazinon;
2-(3-Chlorphenyl)-4-(3-hydroxy-3-methyl-1-butoxy)-5-[4-(aminosulfonyl)phenyl]-3(2H)-pyridazinon;
2-(4-Fluorphenyl)-4-(2-hydroxy-2-methyl-1-propoxy)-5-[4-(methylsulfonyl)phenyl]-3(2H)-pyridazinon;
2-(4-Fluorphenyl)-4-(2-hydroxy-2-methyl-1-propoxy)-5-[4-(aminosulfonyl)phenyl]-3(2H)-pyridazinon;
2-(3-Chlor-4-fluorphenyl)-4-(2-hydroxy-2-methyl-1-propoxy)-5-[4-(methylsulfonyl)phenyl]-3(2H)-pyridazinon;
2-(3-Chlor-4-fluorphenyl)-4-(2-hydroxy-2-methyl-1-propoxy)-5-[4-(aminosulfonyl)phenyl]-3(2H)-pyridazinon;
2-(3-Chlorphenyl)-4-(2-hydroxy-2-methyl-1-propoxy)-5-[4-(methylsulfonyl)phenyl]-3(2H)-pyridazinon;
2-(3-Chlorphenyl)-4-(2-hydroxy-2-methyl-1-propoxy)-5-[4-(aminosulfonyl)phenyl]-3(2H)-pyridazinon;
2-(4-Fluorphenyl)-4-(4-hydroxy-3-methyl-1-butoxy)-5-[4-(aminosulfonyl)phenyl]-3(2H)-pyridazinon;
2-(3,4-Difluorphenyl)-4-(4-hydroxy-3-methyl-1-butoxy)-5-[4-(methylsulfonyl)phenyl]-3(2H)-pyridazinon;
2-(3-Chlor-4-fluorphenyl)-4-(4-hydroxy-3-methyl-1-butoxy)-5-[4-(methylsulfonyl)phenyl]-3(2H)-pyridazinon;
2-(3-Chlorphenyl)-4-(4-hydroxy-3-methyl-1-butoxy)-5-[4-(methylsulfonyl)phenyl]-3(2H)-pyridazinon;
2-(3,4-Difluorphenyl)-4-(4-hydroxy-3-methyl-1-butoxy)-5-[4-(aminosulfonyl)phenyl]-3(2H)-pyridazinon;
2-(3-Chlor-4-fluorphenyl)-4-(4-hydroxy-3-methyl-1-butoxy)-5-[4-(aminosulfonyl)phenyl]-3(2H)-pyridazinon;
2-(3-Chlorphenyl)-4-(4-hydroxy-3-methyl-1-butoxy)-5-[4-(aminosulfonyl)phenyl]-3(2H)-pyridazinon;
(S)-2-(4-Fluorphenyl)-4-(3-hydroxy-2-methyl-1-propoxy)-5-[4-(aminosulfonyl)phenyl]-3(2H)-pyridazinon;
(R)-2-(4-Fluorphenyl)-4-(3-hydroxy-2-methyl-1-propoxy)-5-[4-(aminosulfonyl)phenyl]-3(2H)-pyridazinon;
(S)-2-(3-Chlor-4-fluorphenyl)-4-(3-hydroxy-2-methyl-1-propoxy)-5-[4-(methylsulfonyl)phenyl]-3(2H)-pyridazinon;
(S)-2-(3-Chlor-4-fluorphenyl)-4-(3-hydroxy-2-methyl-1-propoxy)-5-[4-(aminosulfonyl)phenyl]-3(2H)-pyridazinon;
(R)-2-(3-Chlor-4-fluorphenyl)-4-(3-hydroxy-2-methyl-1-propoxy)-5-[4-(methylsulfonyl)phenyl]-3(2H)-pyridazinon;
(R)-2-(3-Chlor-4-fluorphenyl)-4-(3-hydroxy-2-methyl-1-propoxy)-5-[4-(aminosulfonyl)phenyl]-3(2H)-pyridazinon;
(S)-2-(3-Chlorphenyl)-4-(3-hydroxy-2-methyl-1-propoxy)-5-[4-(methylsulfonyl)phenyl]-3(2H)-pyridazinon;
(S)-2-(3-Chlorphenyl)-4-(3-hydroxy-2-methyl-1-propoxy)-5-[4-(aminosulfonyl)phenyl]-3(2H)-pyridazinon;
(R)-2-(3-Chlorphenyl)-4-(3-hydroxy-2-methyl-1-propoxy)-5-[4-(methylsulfonyl)phenyl]-3(2H)-pyridazinon;
(R)-2-(3-Chlorphenyl)-4-(3-hydroxy-2-methyl-1-propoxy)-5-[4-(aminosulfonyl)phenyl]-3(2H)-pyridazinon;
2-(4-Fluorphenyl)-4-(3-hydroxy-2,2-dimethyl-1-propoxy)-5-[4-(methylsulfonyl)phenyl]-3(2H)-pyridazinon;
2-(4-Fluorphenyl)-4-(3-hydroxy-2,2-dimethyl-1-propoxy)-5-[4-(aminosulfonyl)phenyl]-3(2H)-pyridazinon;
2-(3,4-Difluorphenyl)-4-(3-hydroxy-2,2-dimethyl-1-propoxy)-5-[4-(methylsulfonyl)phenyl]-3(2H)-pyridazinon;
2-(3,4-Difluorphenyl)-4-(3-hydroxy-2,2-dimethyl-1-propoxy)-5-[4-(aminosulfonyl)phenyl]-3(2H)-pyridazinon;
2-(3-Chlor-4-fluorphenyl)-4-(3-hydroxy-2,2-dimethyl-1-propoxy)-5-[4-(methylsulfonyl)phenyl]-3(2H)-pyridazinon;
2-(3-Chlor-4-fluorphenyl)-4-(3-hydroxy-2,2-dimethyl-1-propoxy)-5-[4-(aminosulfonyl)phenyl]-3(2H)-pyridazinon;
2-(3-Chlorphenyl)-4-(3-hydroxy-2,2-dimethyl-1-propoxy)-5-[4-(methylsulfonyl)phenyl]-3(2H)-pyridazinon;
2-(3-Chlorphenyl)-4-(3-hydroxy-2,2-dimethyl-1-propoxy)-5-[4-(aminosulfonyl)phenyl]-3(2H)-pyridazinon;
2-(3,4-Dichlorphenyl)-4-(3-hydroxy-3-methyl-1-butoxy)-5-[4-(methylsulfonyl)phenyl]-3(2H)-pyridazinon;
2-[(3-Trifluormethyl)phenyl]-4-(2-hydroxy-2-methyl-1-propoxy)-5-[4-(methylsulfonyl)phenyl]-3(2H)-pyridazinon;
2-(3,4-Dichlorphenyl)-4-(2-hydroxy-2-methyl-1-propoxy)-5-[4-(methylsulfonyl)phenyl]-3(2H)-pyridazinon; und
2-[3-(Trifluormethyl)phenyl]-4-(3-hydroxy-3-methyl-1-butoxy)-5-[4-(methylsulfonyl)phenyl]-3(2H)-pyridazinon;
oder ein pharmazeutisch verträgliches Salz oder Ester davon.

2. Eine Verbindung gewählt aus der Gruppe bestehend aus
2-(2,2,2-Trifluorethyl)-4-(2-hydroxy-2-methyl-1-propoxy)-5-[4-(methylsulfonyl)phenyl]-3(2H)-pyridazinon;
2-(2,2,2-Trifluorethyl)-4-(2-hydroxy-2-methyl-1-propoxy)-5-[4-(aminosulfonyl)phenyl]-3(2H)-pyridazinon;
2-(2,2,2-Trifluorethyl)-4-(3-hydroxy-2,2-dimethyl-1-propoxy)-5-[4-(methylsulfonyl)phenyl]-3(2H)-pyridazinon;
2-(2,2,2-Trifluorethyl)-4-(3-hydroxy-2,2-dimethyl-1-propoxy)-5-[4-(aminosulfonyl)phenyl]-3(2H)-pyridazinon;
(S)-2-(2,2,2-Trifluorethyl)-4-(3-hydroxy-2-methyl-1-propoxy)-5-[4-(methylsulfonyl)phenyl]-3(2H)-pyridazinon;
(S)-2-(2,2,2-Trifluorethyl)-4-(3-hydroxy-2-methyl-1-propoxy)-5-[4-(aminosulfonyl)phenyl]-3(2H)-pyridazinon;
(R)-2-(2,2,2-Trifluorethyl)-4-(3-hydroxy-2-methyl-1-propoxy)-5-[4-(methylsulfonyl)phenyl]-3(2H)-pyridazinon;
(R)-2-(2,2,2-Trifluorethyl)-4-(3-hydroxy-2-methyl-1-propoxy)-5-[4-(aminosulfonyl)phenyl]-3(2H)-pyridazinon;
2-(2,2,2-Trifluorethyl)-4-(3-hydroxy-3-methyl-1-butoxy)-5-[4-(methylsulfonyl)phenyl]-3(2H)-pyridazinon; und
2-(2,2,2-Trifluorethyl)-4-(3-hydroxy-3-methyl-1-butoxy)-5-[4-(aminosulfonyl)phenyl]-3(2H)-pyridazinon;
oder ein pharmazeutisch verträgliches Salz oder Ester davon.

3. Eine Verbindung gewählt aus der Gruppe bestehend aus
N-[[4-[2-(4-Fluorphenyl)-4-(3-hydroxy-3-methyl-1-butoxy)-2H-pyridazin-3-on-5-yl]phenyl]sulfonyl]acetamid;
N-[[4-[2-(4-Fluorphenyl)-4-(3-hydroxy-3-methyl-1-butoxy)-2H-pyridazin-3-on-5-yl]phenyl]sulfonyl]acetamid, Natriumsalz;
N-[[4-[2-(3,4-Difluorphenyl)-4-(2-hydroxy-2-methyl-1-propoxy)-2H-pyridazin-3-on-5-yl]phenyl]sulfonyl]acetamid;
N-[[4-[2-(3,4-Difluorphenyl)-4-(2-hydroxy-2-methyl-1-propoxy)-2H-pyridazin-3-on-5-yl]phenyl]sulfonyl]acetamid, Natriumsalz;
N-[[4-[2-(3-Chlor-4-fluorphenyl)-4-(2-hydroxy-2-methyl-1-propoxy)-2H-pyridazin-3-on-5-yl]phenyl]sulfonyl]acetamid;
N-[[4-[2-(3-Chlor-4-fluorphenyl)-4-(2-hydroxy-2-methyl-1-propoxy)-2H-pyridazin-3-on-5-yl]phenyl]sulfonyl]acetamid, Natriumsalz;
N-[[4-[2-(3-Chlorphenyl)-4-(2-hydroxy-2-methyl-1-propoxy)-2H-pyridazin-3-on-5-yl]phenyl]sulfonyl]acetamid;
N-[[4-[2-(3-Chlorphenyl)-4-(2-hydroxy-2-methyl-1-propoxy)-2H-pyridazin-3-on-5-yl]phenyl]sulfonyl]acetamid, Natriumsalz;
N-[[4-[2-(2,2,2-Trifluorethyl)-4-(2-hydroxy-2-methyl-1-propoxy)-2H-pyridazin-3-on-5-yl]phenyl]sulfonyl]acetamid;
N-[[4-[2-(2,2,2-Trifluorethyl)-4-(2-hydroxy-2-methyl-1-propoxy)-2H-pyridazin-3-on-5-yl]phenyl]sulfonyl]acetamid, Natriumsalz;
N-[[4-[2-(2,2,2-Trifluorethyl)-4-(3-hydroxy-2,2-dimethyl-1-propoxy)-2H-pyridazin-3-on-5-yl]phenyl]sulfonyl]acetamid; und
N-[[4-[2-(2,2,2-Trifluorethyl)-4-(3-hydroxy-2,2-dimethyl-1-propoxy)-2H-pyridazin-3-on-5-yl]phenyl]sulfonyl]acetamid Natriumsalz;
oder ein pharmazeutisch verträgliches Salz oder Ester davon.

4. 2-(3,4-Difluorphenyl)-4-(3-hydroxy-3-methyl-1-butoxy)-5-[4-(methylsulfonyl)phenyl]-3(2H)-pyridazinon oder ein pharmazeutisch verträgliches Salz oder Ester davon.

5. Eine pharmazeutische Zusammensetzung, die eine therapeutisch wirksame Menge einer Verbindung von irgendeinem der Ansprüche 1-4 und einen pharmazeutisch verträglichen Träger umfaßt.

6. Eine Verbindung von irgendeinem der Ansprüche 1-4 für die Verwendung als ein therapeutischer Wirkstoff.

7. Verwendung einer Verbindung von irgendeinem der Ansprüche 1-4 zur Herstellung eines Medikaments für die Hemmung der Prostaglandinbiosynthese in einem Säugetier, das eine solche Behandlung benötigt.

8. Verwendung einer Verbindung von irgendeinem der Ansprüche 1-4 zur Herstellung eines Medikaments für die Behandlung von Schmerzen, Fieber, Entzündung, rheumatoider Arthritis, Osteoarthritis, Adhäsionen und Krebs.

## Revendications

1. Composé choisi dans le groupe consistant en
2-(4-fluorophényl)-4-(4-hydroxy-2-méthyl-1-butoxy)-5-[4-(méthylsulfonyl)phényl]-3(2H)-pyridazinone ;
2-(4-fluorophényl)-4-(3-hydroxy-3-méthyl-1-butoxy)-5-[4-(aminosulfonyl)-phényl]-3(2H)-pyridazinone ;
2-(3,4-difluorophényl)-4-(3-hydroxy-3-méthyl-1-butoxy)-5-[4-(méthylsulfonyl)phényl]-3(2H)-pyridazinone ;
2-(3,4-difluorophényl)-4-(3-hydroxy-3-méthyl-1-butoxy)-5-[4-(amino-sulfonyl)phényl]-3(2H)-pyridazinone ;
2-(3-chloro-4-fluorophényl)-4-(3-hydroxy-3-méthyl-1-butoxy)-5-[4-(méthylsulfonyl)phényl]-3(2H)-pyridazinone ;
2-(3-chloro-4-fluorophényl)-4-(3-hydroxy-3-méthyl-1-butoxy)-5-[4-(amino-sulfonyl)phényl]-3(2H)-pyridazinone ;
2-(3-chlorophényl)-4-(3-hydroxy-3-méthyl-1-butoxy)-5-[4-(méthylsulfonyl)phényl]-3(2H)-pyridazinone ;
2-(3-chlorophényl)-4-(3-hydroxy-3-méthyl-1-butoxy)-5-[4-(amino-sulfonyl)phényl]-3(2H)-pyridazinone ;
2-(4-fluorophényl)-4-(2-hydroxy-2-méthyl-1-propoxy)-5-[4-(méthylsulfonyl)phényl]-3(2H)-pyridazinone ;
2-(4-fluorophényl)-4-(2-hydroxy-2-méthyl-1-propoxy)-5-[4-(amino-sulfonyl)phényl]-3(2H)-pyridazinone ;
2-(3-chloro-4-fluorophényl)-4-(2-hydroxy-2-méthyl-1-propoxy)-5-[4-(méthylsulfonyl)phényl]-3(2H)-pyridazinone ;
2-(3-chloro-4-fluorophényl)-4-(2-hydroxy-2-méthyl-1-propoxy)-5-[4-(amino-sulfonyl)phényl]-3(2H)-pyridazinone ;
2-(3-chlorophényl)-4-(2-hydroxy-2-méthyl-1-propoxy)-5-[4-(méthylsulfonyl)phényl]-3(2H)-pyridazinone ;
2-(3-chlorophényl)-4-(2-hydroxy-2-méthyl-1-propoxy)-5-[4-(amino-sulfonyl)phényl]-3(2H)-pyridazinone ;
2-(4-fluorophényl)-4-(4-hydroxy-3-méthyl-1-butoxy)-5-[4-(amino-sulfonyl)phényl]-3(2H)-pyridazinone ;
2-(3,4-difluorophényl)-4-(4-hydroxy-3-méthyl-1-butoxy)-5-[4-(méthylsulfonyl)phényl]-3(2H)-pyridazinone ;
2-(3-chloro-4-fluorophényl)-4-(4-hydroxy-3-méthyl-1-butoxy)-5-[4-(méthylsulfonyl)phényl]-3(2H)-pyridazinone ;
2-(3-chlorophényl)-4-(4-hydroxy-3-méthyl-1-butoxy)-5-[4-(méthylsulfonyl)phényl]-3(2H)-pyridazinone ;
2-(3,4-difluorophényl)-4-(4-hydroxy-3-méthyl-1-butoxy)-5-[4-(amino-sulfonyl)phényl]-3(2H)-pyridazinone ;
2-(3-chloro-4-fluorophényl)-4-(4-hydroxy-3-méthyl-1-butoxy)-5-[4-(amino-sulfonyl)phényl]-3(2H)-pyridazinone ;
2-(3-chlorophényl)-4-(4-hydroxy-3-méthyl-1-butoxy)-5-[4-(amino-sulfonyl)phényl]-3(2H)-pyridazinone ;
(S)-2-(4-fluorophényl)-4-(3-hydroxy-2-méthyl-1-propoxy)-5-[4-(amino-sulfonyl)phényl]-3(2H)-pyridazinone ;
(R)-2-(4-fluorophényl)-4-(3-hydroxy-2-méthyl-1-propoxy)-5-[4-(amino-sulfonyl)phényl]-3(2H)-pyridazinone ;
(S)-2-(3-chloro-4-fluorophényl)-4-(3-hydroxy-2-méthyl-1-propoxy)-5-[4-(méthylsulfonyl)phényl]-3(2H)-pyridazinone ;
(S)-2-(3-chloro-4-fluorophényl)-4-(3-hydroxy-2-méthyl-1-propoxy)-5-[4-(aminosulfonyl)phényl]-3(2H)-pyridazinone ;
(R)-2-(3-chloro-4-fluorophényl)-4-(3-hydroxy-2-méthyl-1-propoxy)-5-[4-(méthylsulfonyl)phényl]-3(2H)-pyridazinone ;
(R)-2-(3-chloro-4-fluorophényl)-4-(3-hydroxy-2-méthyl-1-propoxy)-5-[4-(aminosulfonyl)phényl]-3(2H)-pyridazinone ;
(S)-2-(3-chlorophényl)-4-(3-hydroxy-2-méthyl-1-propoxy)-5-[4-(méthylsulfonyl)phényl]-3(2H)-pyridazinone ;
(S)-2-(3-chlorophényl)-4-(3-hydroxy-2-méthyl-1-propoxy)-5-[4-(amino-sulfonyl)phényl]-3(2H)-pyridazinone ;
(R)-2-(3-chlorophényl)-4-(3-hydroxy-2-méthyl-1-propoxy)-5-[4-(méthylsulfonyl)phényl]-3(2H)-pyridazinone ;
(R)-2-(3-chlorophényl)-4-(3-hydroxy-2-méthyl-1-propoxy)-5-[4-(amino-sulfonyl)phényl]-3(2H)-pyridazinone ;
2-(4-fluorophényl)-4-(3-hydroxy-2,2-diméthyl-1-propoxy)-5-[4-(méthylsulfonyl)phényl]-3(2H)-pyridazinone ;
2-(4-fluorophényl)-4-(3-hydroxy-2,2-diméthyl-1-propoxy)-5-[4-(amino-sulfonyl)phényl]-3(2H)-pyridazinone ;
2-(3,4-difluorophényl)-4-(3-hydroxy-2,2-diméthyl-1-propoxy)-5-[4-(méthylsulfonyl)phényl]-3(2H)-pyridazinone ;
2-(3,4-difluorophényl)-4-(3-hydroxy-2,2-diméthyl-1-propoxy)-5-[4-(aminosulfonyl)phényl]-3(2H)-pyridazinone ;
2-(3-chloro-4-fluorophényl)-4-(3-hydroxy-2,2-diméthyl-1-propoxy)-5-[4-(méthylsulfonyl)phényl]-3(2H)-pyridazinone ;
2-(3-chloro-4-fluorophényl)-4-(3-hydroxy-2,2-diméthyl-1-propoxy)-5-[4-(aminosulfonyl)phényl]-3(2H)-pyridazinone ;
2-(3-chlorophényl)-4-(3-hydroxy-2,2-diméthyl-1-propoxy)-5-[4-(méthylsulfonyl)phényl]-3(2H)-pyridazinone ;
2-(3-chlorophényl)-4-(3-hydroxy-2,2-diméthyl-1-propoxy)-5-[4-(amino-sulfonyl)phényl]-3(2H)-pyridazinone ;
2-(3,4-dichlorophényl)-4-(3-hydroxy-3-méthyl-1-butoxy)-5-[4-(méthylsulfonyl)phényl]-3(2H)-pyridazinone ;
2-[(3-trifluorométhyl)phényl]-4-(2-hydroxy-2-méthyl-1-propoxy)-5-[4-(méthyl-sulfonyl)phényl]-3(2H)-pyridazinone ;
2-(3,4-dichlorophényl)-4-(2-hydroxy-2-méthyl-1-propoxy)-5-[4-(méthylsulfonyl)phényl]-3(2H)-pyridazinone ; et
2-[3-(trifluorométhyl)phényl]-4-(3-hydroxy-3-méthyl-1-butoxy)-5-[4-(méthylsulfonyl)phényl]-3(2H)-pyridazinone;
ou sel ou ester pharmaceutiquement acceptable de celui-ci.

2. Composé choisi dans le groupe consistant en
2-(2,2,2-trifluoroéthyl)-4-(2-hydroxy-2-méthyl-1-propoxy)-5-[4-(méthylsulfonyl)phényl]-3(2H)-pyridazinone ;
2-(2,2,2-trifluoroéthyl)-4-(2-hydroxy-2-méthyl-1-propoxy)-5-[4-(amino-sulfonyl)phényl]-3(2H)-pyridazinone ;
2-(2,2,2-trifluoroéthyl)-4-(3-hydroxy-2,2-diméthyl-1-propoxy)-5-[4-(méthylsulfonyl)phényl]-3(2H)-pyridazinone ;
2-(2,2,2-trifluoroéthyl)-4-(3-hydroxy-2,2-diméthyl-1-propoxy)-5-[4-(aminosulfonyl)phényl]-3(2H)-pyridazinone ;
(S)-2-(2,2,2-trifluoroéthyl)-4-(3-hydroxy-2-méthyl-1-propoxy)-5-[4-(méthylsulfonyl)phényl]-3(2H)-pyridazinone ;
(S)-2-(2,2,2-trifluoroéthyl)-4-(3-hydroxy-2-méthyl-1-propoxy)-5-[4-(amino-sulfonyl)phényl]-3(2H)-pyridazinone ;
(R)-2-(2,2,2-trifluoroéthyl)-4-(3-hydroxy-2-méthyl-1-propoxy)-5-[4-(méthylsulfonyl)phényl]-3(2H)-pyridazinone ;
(R)-2-(2,2,2-trifluoroéthyl)-4-(3-hydroxy-2-méthyl-1-propoxy)-5-[4-(aminosulfonyl)phényl]-3(2H)-pyridazinone ;
2-(2,2,2-trifluoroéthyl)-4-(3-hydroxy-3-méthyl-1-butoxy)-5-[4-(méthylsulfonyl)phényl]-3(2H)-pyridazinone ; et
2-(2,2,2-trifluoroéthyl)-4-(3-hydroxy-3-méthyl-1-butoxy)-5-[4-(amino-sulfonyl)phényl]-3(2H)-pyridazinone ;
ou sel ou ester pharmaceutiquement acceptable de celui-ci.

3. Composé choisi dans le groupe consistant en
N-[[4-[2-(4-fluorophényl)-4-(3-hydroxy-3-méthyl-1-butoxy)-2H-pyridazin-3-on-5-yl]phényl]sulfonyl]acétamide ;
N-[[4-[2-(4-fluorophényl)-4-(3-hydroxy-3-méthyl-1-butoxy)-2H-pyridazin-3-on-5-yl]phényl]sulfonyl]acétamide, sel de sodium ;
N-[[4-[2-(3,4-difluorophényl)-4-(2-hydroxy-2-méthyl-1-propoxy)-2H-pyridazin-3-on-5-yl]phényl]sulfonyl]acétamide ;
N-[[4-[2-(3,4-difluorophényl)-4-(2-hydroxy-2-méthyl-1-propoxy)-2H-pyridazin-3-on-5-yl]phényl]sulfonyl]acétamide, sel de sodium ;
N-[[4-[2-(3-chloro-4-fluorophényl)-4-(2-hydroxy-2-méthyl-1-propoxy)-2H-pyridazin-3-on-5-yl]phényl]sulfonyl]acétamide ;
N-[[4-[2-(3-chloro-4-fluorophényl)-4-(2-hydroxy-2-méthyl-1-propoxy)-2H-pyridazin-3-on-5-yl]phényl]sulfonyl]acétamide, sel de sodium ;
N-[[4-[2-(3-chlorophényl)-4-(2-hydroxy-2-méthyl-1-propoxy)-2H-pyridazin-3-on-5-yl]phényl]sulfonyl]acétamide ;
N-[[4-[2-(3-chlorophényl)-4-(2-hydroxy-2-méthyl-1-propoxy)-2H-pyridazin-3-on-5-yl]phényl]sulfonyl]acétamide, sel de sodium ;
N-[[4-[2-(2,2,2-trifluoroéthyl)-4-(2-hydroxy-2-méthyl-1-propoxy)-2H-pyridazin-3-on-5-yl]phényl]sulfonyl]acétamide ;
N-[[4-[2-(2,2,2-trifluoroéthyl)-4-(2-hydroxy-2-méthyl-1-propoxy)-2H-pyridazin-3-on-5-yl]phényl]sulfonyl]acétamide, sel de sodium ;
N-[[4-[2-(2,2,2-trifluoroéthyl)-4-(3-hydroxy-2,2-diméthyl-1-propoxy)-2H-pyridazin-3-on-5-yl]phényl]sulfonyl]acétamide ; et
N-[[4-[2-(2,2,2-trifluoroéthyl)-4-(3-hydroxy-2,2-diméthyl-1-propoxy)-2H-pyridazin-3-on-5-yl]phényl]sulfonyl]acétamide, sel de sodium ;
ou sel ou ester pharmaceutiquement acceptable de celui-ci.

4. 2-(3,4-difluorophényl)-4-(3-hydroxy-3-méthyl-1-butoxy)-5-[4-(méthylsulfonyl)phényl]-3(2H)-pyridazinone ou sel ou ester pharmaceutiquement acceptable de celle-ci.

5. Composition pharmaceutique comprenant une quantité thérapeutiquement efficace d'un composé selon l'une quelconque des revendications 1-4 et un support pharmaceutiquement acceptable.

6. Composé selon l'une quelconque des revendications 1-4 destiné à être utilisé comme agent thérapeutique.

7. Utilisation d'un composé selon l'une quelconque des revendications 1-4 pour fabriquer un médicament pour inhiber la biosynthèse de prostaglandines chez un mammifère qui nécessite un tel traitement.

8. Utilisation d'un composé selon l'une quelconque des revendications 1-4 pour fabriquer un médicament pour traiter la douleur, la fièvre, l'inflammation, la polyarthrite rhumatoïde, l'arthrose, les adhésions et le cancer.
